# EUROPEAN PATENT SPECIFICATION

(11) **EP 4 103 081 B1**
(45) Date of publication and mention of the grant of the patent: **04.11.2026**
(21) Application number: 21753531.9
(22) Date of filing: 12.02.2021
(51) Int. Cl.: A61B 17/34, A61M 25/06

(54) **OBTURATOR FOR CANNULA WITH INTERNAL FEATURES**
OBTURATOR FÜR KANÜLE MIT INNEREN MERKMALEN
OBTURATEUR POUR CANULE À CARACTÉRISTIQUES INTERNES

(30) Priority: 14.02.2020 US 202062976991 P
(43) Date of publication of application: 21.12.2022
(73) Proprietor: Fisher & Paykel Healthcare Limited, East Tamaki Auckland 2013 (NZ)
(72) Inventor: SMITH, Daniel Joel, Auckland, 2013 (NZ); KURIGER, Donald Roy, Auckland, 2013 (NZ); FREEBAIRN, Scott Hamish, Auckland, 2013 (NZ); GREENFIELD, James Robert Jarmey, Auckland, 2013 (NZ); BAUMANN, Monika, Auckland, 2013 (NZ); LEE, Joshua Robert, Auckland, 2013 (NZ); HAZARD, Stephanie Louise, Auckland, 2013 (NZ); KOKHANENKO, Pavlo, Auckland, 2013 (NZ); WARNER, Zach Jonathan, Auckland, 2013 (NZ); ARULANDU, Abigail Sharmini Rajen, Auckland, 2013 (NZ); AMADOR NORIEGA, Jesus Antonio, Auckland, 2013 (NZ); REVELLY, Simon, Auckland, 2013 (NZ); PEGMAN, Benjamin Elliot Hardinge, Auckland, 2013 (NZ); LAUS, Charlotte Grace, Auckland, 2013 (NZ); STREVENS, Joseph Patrick Walter, Auckland, 2013 (NZ); VAN DEN HEUIJ, Lotte Gertrudis Theodora, Auckland, 2013 (NZ); TEH, Eu-Lee, Auckland, 2013 (NZ); MONRO, Rory Alexander, Auckland, 2013 (NZ); GILBERT, James Michael, Auckland, 2013 (NZ)
(74) Representative: Haseltine Lake Kempner LLP
(86) International application number: PCT/IB2021/051151
(87) International publication number: WO 2021/161228

(56) References cited:
- WO-A1-2006/103408
- WO-A1-2010/048965
- US-A- 5 746 720
- US-A1- 2005 090 852
- US-A1- 2009 018 394
- US-A1- 2010 081 988
- US-A1- 2010 222 801
- US-A1- 2011 087 159
- US-A1- 2013 023 733
- US-A1- 2014 249 371
- US-A1- 2014 323 807
- US-A1- 2014 323 808
- US-A1- 2015 038 994
- US-A1- 2016 270 815
- US-A1- 2017 215 916
- US-A1- 2018 140 286
- US-B1- 6 228 063

## Description

### FIELD OF THE DISCLOSURE

The present disclosure relates in some aspects to an obturator configured to be used with a cannula having internal features, in particular during a medical procedure.

### BACKGROUND

Various medical procedures require the provision of gases, typically carbon dioxide, to a patient during the medical procedure. For example, two general categories of medical procedures often require providing gases to a patient. These include closed type medical procedures and open type medical procedures.

In closed type medical procedures, an insufflator is arranged to deliver gases to a body cavity of the patient to inflate the body cavity and/or to resist collapse of the body cavity during the medical procedure. Examples of such medical procedures include laparoscopy and endoscopy, although an insufflator may be used with any other type of medical procedure as required. Endoscopic procedures enable a medical practitioner to visualize a body cavity by inserting an endoscope or the like through one or more natural openings, small puncture(s), or incision(s) to generate an image of the body cavity. In laparoscopy procedures, a medical practitioner typically inserts a medical instrument through natural openings, small puncture(s), or incision(s) to perform a medical procedure in the body cavity. In some cases, an initial endoscopic procedure may be carried out to assess the body cavity, and then a subsequent laparoscopy carried out to operate on the body cavity. Such procedures are widely used, for example, on the peritoneal cavity, or during a thoracoscopy, colonoscopy, gastroscopy or bronchoscopy.

Medical instruments, including apparatus for delivering gases and to visualize the body cavity can be inserted into the body cavity before and during these medical procedures. Devices can be used to create one or more entry ports for delivery of gases and medical instrument access. Obturators can be used to assist insertion of a cannula into a body cavity.

Various techniques are known in surgery for gaining entry to the body cavity. An open entry technique involves creating a wound by cutting through the tissue until the site of interest is reached. A blunt obturator is assembled into a cannula and this assembly is inserted into the wound. The cannula is retained in the wound, typically with an outer sliding retention device and the obturator is removed.

An optical entry technique uses an optical obturator in a cannula as a trocar assembly. The optical obturator has a lumen into which a surgical scope can be inserted, and an optical clear tip to allow the scope to view through the tip of the obturator. A small incision is made in the skin and the trocar assembly is inserted into the wound. The trocar is pushed through the tissue until the site of interest is reached. Using the scope viewing through the tip of the obturator, the depth of the trocar in the tissue can be determined, mitigating risk of inserting the trocar too far into the tissue. The obturator with the scope is then removed and the scope can be inserted into the remaining cannula while the patient is insufflated to visualize the body cavity.

A closed entry technique utilizes an obturator with a dilating or bladed tip. An incision is made in the skin then skin around the wound is lifted and the obturator and cannula are pushed through. Alternatively, the site of interest can be insufflated first using a Veress needle. Once the body cavity is inflated, the skin is held while a bladed or dilating obturator and cannula are pushed into the body cavity. The obturator is then removed from the cannula.

The obturator can be used together with the cannula for insertion into the body cavity. Some cannulas can have internal structures (such as ribs or guide elements) in the inner lumen or other internal surface of the cannula. It is desirable that the internal structures present on the inside or inner lumen of cannula are not damaged during use of the obturator, both for purposes of maintaining their function and also to avoid internal cannula components from dislodging and entering the body cavity.

US 2009/018394 A1, US 6 228 063 B1, US 2013/023733 A1 and US 2014/249371 A1 disclose trocar assemblies of the prior art.

### SUMMARY

The invention is defined by independent claim 1, with further embodiments defined by the dependent claims.

The present disclosure provides examples of an obturator configured to accommodate a cannula with internal structural features or elements.

Structural elements can be added or provided inside the cannula for various reasons, such as, but not limited to, directing gas flow to the surgical cavity utilizing a directed gas flow cannula. These structural elements in the cannula, which protrude inwardly into the cannula lumen, may interfere with or impede the use of an obturator in a trocar assembly. The obturator may damage or break off such structural elements, impeding their intended function and/or causing pieces of the cannula to break off and potentially enter the body cavity. The present disclosure provides examples of an obturator and/or a trocar assembly that seeks to remedy or ameliorate the aforementioned problems and/or other problems (including, for example, preventing the cannula elements from interfering or impeding the use of the obturator for entry techniques).

In some cases, an obturator for use in a trocar assembly can comprise an obturator body, an obturator shaft extending from the body and a penetrating end at a distal end thereof. The obturator shaft can be at least partially positionable within a cannula and can comprise one or more internal structure accommodating portions, configured to locate around or adjacent to one or more internal structures located in and/or on an interior wall of a shaft of the cannula.

The obturator described in any of the preceding paragraphs may further comprise one or more of the following features. The one or more internal structure accommodating portions can comprise one or more recesses, slots or openings located in a surface of the obturator shaft. The one or more internal structure accommodating portions can comprise a portion of the obturator shaft comprising a reduced diameter relative to the diameter of the remainder of the obturator shaft. The one or more internal structure accommodating portions can comprise a deformable material disposed around a portion of the obturator shaft, wherein the deformable material deforms/flexes around the internal structures on the cannula when the obturator is inserted in the cannula. The one or more internal structure accommodating portions can be located at or adjacent to the distal end of the obturator shaft. The one or more recesses or openings can provide clearance for the obturator from the internal structures on the cannula and may also facilitate ease of insertion and placement of the obturator inside the cannula. The one or more recesses or openings can extend from the distal end of the obturator shaft, extending at least partway towards the obturator body. The deformable material can be configured as a sheath around the distal end of the obturator shaft. The deformable material can be provided as a ring around the distal end of the obturator shaft. The ring can deform when the obturator slides within the cannula and spring open on exiting the open distal end of the cannula to prevent debris from entering the cannula/sticking to internal structures.

The obturator body can have one or more locating elements that can be configured to mate with one or more corresponding receiving elements in the cannula. The one or more locating elements can comprise one or more pins extending from an underside of the obturator body. The one or more locating elements can orient the obturator to the cannula before the cannula internal structures contact the obturator. The one or more locating elements can include at least one locking finger extending outwardly or distally from a lower surface of the obturator body, the at least one locking finger configured to be received in a corresponding aperture in the cannula. The locking finger can comprise a locking tab configured to engage with a corresponding engagement tab in the cannula.

The obturator can be an optical obturator, comprising a lumen configured to receive a scope. The obturator body can have one or more seal elements configured to prevent gas leaking from the obturator lumen. The one or more seal elements can comprise an upper seal configured to seal around a medical instrument when inserted into the obturator lumen and a lower seal configured to create a gas seal when there is no medical instrument in the obturator lumen. The one or more seal elements can be located about the proximal open end of the obturator body, the one or more seal elements configured to prevent gas leaking out of the obturator body. The one or more seal elements can be a flexible or deformable material which provide a seal against the medical instrument when inserted into the obturator. In this application, reference to a medical instrument may include an endoscope or camera, laparoscope or other scope device. The flexible or deformable material can fit over a top surface of the obturator body. The penetrating end of the obturator can have a blunt or dilating tip. The tip can be bladed or unbladed.

In some cases, a trocar assembly can comprise a cannula comprising a shaft with a lumen, and one or more internal structures disposed on at least a portion of the lumen; and an obturator, the obturator comprising an obturator shaft with one or more internal structure accommodating portions configured to locate around or adjacent to the one or more internal structures on the cannula lumen when the obturator shaft is located in the lumen of the cannula.

The trocar assembly described in any of the preceding paragraphs may further comprise one or more of the following features. The obturator can further comprise an obturator body, an obturator shaft extending from the obturator body and a penetrating end at a distal end thereof. The obturator shaft can comprise one or more internal structure accommodating portions, configured to locate around or adjacent to the one or more internal structures on the cannula lumen.

The one or more internal structure accommodating portions can comprise one or more recesses or openings located in a surface of the obturator shaft. The one or more internal structure accommodating portions can comprise a portion of the obturator shaft comprising a reduced diameter relative to the diameter of the remainder of the obturator shaft. The one or more internal structure accommodating portions can comprise a deformable material disposed at least partly around a portion of the obturator shaft, wherein the deformable material deforms/flexes around the one or more internal structures when the obturator is inserted in the cannula. The one or more internal structure accommodating portions can be located at or adjacent to the distal end of the obturator shaft.

The one or more recesses or openings can provide clearance for the obturator from the internal structures on the cannula. The one or more recesses or openings can extend from the distal end of the obturator shaft, extending at least partway towards the obturator body. The deformable material can be configured as a sheath around the distal end of the obturator shaft. The deformable material can be provided as a ring around the distal end of the obturator shaft. The ring can deform when the obturator slides within the cannula and springs open on exiting the open distal end of cannula to prevent debris from entering the cannula/sticking to internal structures.

The obturator body can have one or more locating elements configured to mate with one or more corresponding receiving element in the cannula. The one or more locating elements can comprise one or more pins extending from an underside of the obturator body. The one or more locating elements can orient the obturator to the cannula before the one or more internal structures protruding outwardly from a surface of the lumen of the cannula contact the obturator. The one or more locating elements can include at least one locking finger extending outwardly from lower surface of the obturator body, the at least one locking finger configured to be received in a corresponding aperture in the cannula. The at least one locking finger can comprise a locking tab configured to engage with corresponding engagement tab in the cannula.

The obturator can be an optical obturator, comprising a lumen configured to receive a scope. The obturator body can have one or more seal elements configured to prevent gas leaking from the obturator lumen. The one or more seal elements can comprise an upper seal configured to seal about a scope when inserted into the obturator lumen and a lower seal configured to create a gas seal when there is no scope in the obturator lumen. The one or more seal elements can be located about the proximal open end of the obturator body, the seal elements configured to prevent gas leaking out of the obturator body. The one or more seal elements can be a flexible or deformable material which provide a seal against the scope when inserted into the obturator. The flexible or deformable material can fit over a top surface of the obturator body.

The penetrating end of the obturator can have a blunt or dilating tip. The tip can be bladed or unbladed. The trocar assembly can be configured to be used with a humidifier and/or insufflator system to supply gases to a patient, and/or with other medical instruments, such as those typically used in laparoscopic medical procedures.

### BRIEF DESCRIPTION OF THE DRAWINGS

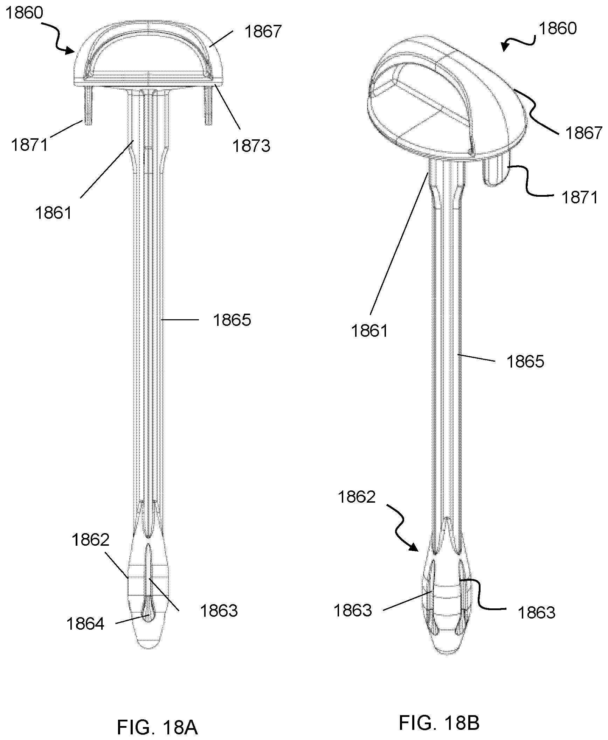

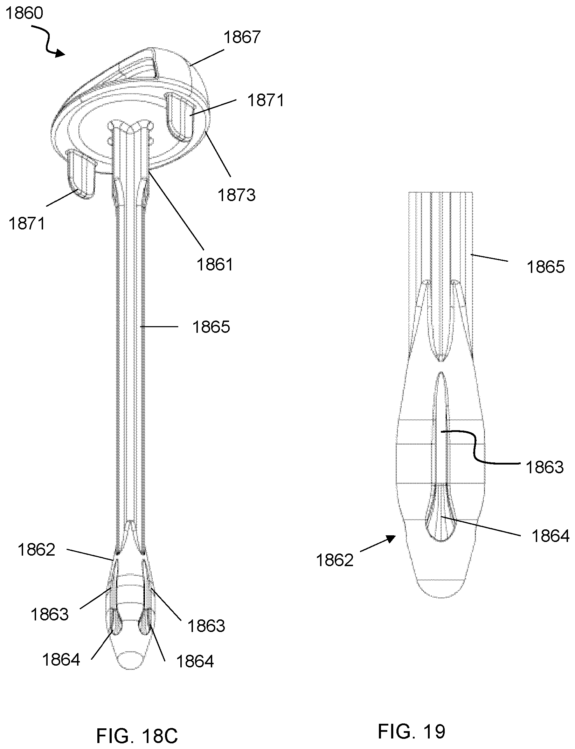

These and other features, aspects, and advantages of the present disclosure are described with reference to the drawings of certain embodiments, which are intended to schematically illustrate certain embodiments and not to limit the disclosure. In some cases, a "slice" has been shown for clarity purposes for some sectional and cross-sectional views of a three-dimensional cannula. A person reasonably skilled in the art would be able to appreciate that these figures illustrate a slice of a three-dimensional cannula. In some cases, the projection surfaces have not been shown for clarity. For example, projecting hole surfaces have not been shown in some views. Figures 18A-20 depict embodiments and/or aspects of the invention.
Figure 1 illustrates schematically an example medical gases delivery apparatus in use in surgery.
Figure 2 illustrates schematically an example medical gases delivery apparatus.
Figures 3A-3B illustrate various views of an embodiment of a cannula with a directed gas flow lumen and guide elements.
Figure 4 illustrates a view of an embodiment of a cannula with a directed gas flow lumen and guide elements.
Figures 5A-5P illustrate embodiments of obturator tip variations that can be used with a directed gases flow cannula.
Figures 6A-6B illustrate views of an embodiment of an obturator within a cannula with internal structures.
Figures 6C-6D illustrate views of the obturator of Figures 6A-6B.
Figures 7A-7B illustrate an embodiment of an obturator within a cannula with internal structures.
Figures 7C-7D are views of the obturator of Figures 7A-7B.
Figures 8A-8D illustrate views of embodiments of an obturator with a reduced diameter portion.
Figures 9A-9D illustrate views of an embodiment of an obturator with a flexible or deformable material in the obturator shaft and/or tip.
Figures 10A-10D illustrate views of an embodiment of the obturator with a flexible ring around the shaft and/or tip.
Figures 11A-11E illustrate views of an obturator with locating elements for aligning and orienting the obturator in a cannula.
Figures 12A-12E illustrate an obturator with locating elements to align and orient the obturator in a cannula.
Figures 13A-13E illustrate views of an embodiment of the obturator with one or more seal elements.
Figures 14A-14E illustrate views of an embodiment of an obturator with one or more proximal seals.
Figure 15 illustrates an embodiment of a handle on an obturator.
Figures 16A-16C illustrate an embodiment of an obturator with elongated internal structures.
Figure 17 illustrates an enlarged view of the obturator distal tip of Figures 16A - 16C.
Figures 18A-18C illustrate an embodiment of an obturator with flared elongated internal structures.
Figure 19 illustrates an enlarged view of the obturator distal tip of Figures 18A - 18C.
Figure 20 illustrates the obturator of Figures 18A-18C inserted inside a cannula to form a trocar.

### DETAILED DESCRIPTION

Although certain embodiments and examples are described below, those of skill in the art will appreciate that the disclosure extends beyond the specifically disclosed embodiments and/or uses and obvious modifications and equivalents thereof. Thus, it is intended that the scope of the disclosure herein disclosed should not be limited by any particular embodiments described below.

### Example Medical Gases Delivery Systems

Fluids, for example, gases can be introduced to a surgical cavity, for example, the peritoneal cavity via a cannula inserted through an incision made in a patient's body (for example, the abdominal wall). A trocar can be used to create an entry point and/or to insert the cannula into the patient's body. The trocar can include an obturator and a cannula. The cannula and obturator can be used in creating and/or maintaning a medical instrument entry port. The obturator can fit down the bore or inner lumen of the cannula to create the entry port. As described herein, structures such as guide elements may be present on the inner wall of the cannula. These structures or guide elements can interfere with ordinary use of an obturator yet need to remain intact throughout the medical procedure to achieve their intended purpose.

The cannula can be coupled to an insufflator. The gases flow from the insufflator can be provided to inflate the surgical cavity (for example, to maintain a pneumoperitoneum, which is a cavity filled with gas within the abdomen). A medical instrument can be inserted through the cannula into the inflated surgical cavity. For example, an endoscope, another vision system, including but not limited to a scope, or a camera unit can be inserted into the cavity. Visibility in the cavity can be assisted by introduction of fluid (gas or liquid), which can be air, carbon dioxide, saline, or any other suitable gas or liquid. In some cases, while the term gas or gases can be used to refer to the fluid that is inserted through the cannula and/or into the cavity as described herein, it is understood that any fluid, including any gas or liquid, can be used. After initial insufflation and insertion of the instrument (for example, a laparoscope) through the cannula, additional cannulas can be placed in the surgical cavity under laparoscopic observation. A trocar can be used to create and/or maintain the entry ports. At the end of the operating procedure, all instruments and cannulas are removed from the surgical cavity, the gases are expelled, and each incision is closed. In some embodiments, the pressure within the surgical cavity is maintained to be substantially constant by the insufflator. The system can also include one or more vents to remove (e.g., vent out) smoke and gases from the cavity. The vent may in some cases include a tap that is manually activated by a medical professional. The insufflator may be controlled to compensate for reduction in pressure during venting, e.g., via controlling delivery of fresh insufflation gases into the cavity. For thoracoscopy, colonoscopy, sigmoidoscopy, gastroscopy, bronchoscopy, and/or others, the same or substantially similar procedure for introducing gases to a surgical cavity can be followed. The quantity and flow of gases can be controlled by the clinician performing the examination and/or automatically by the surgical system.

In some figures described herein, a slice of the trocar, cannula, and/or obturator can be shown as opposed to a cross-section.

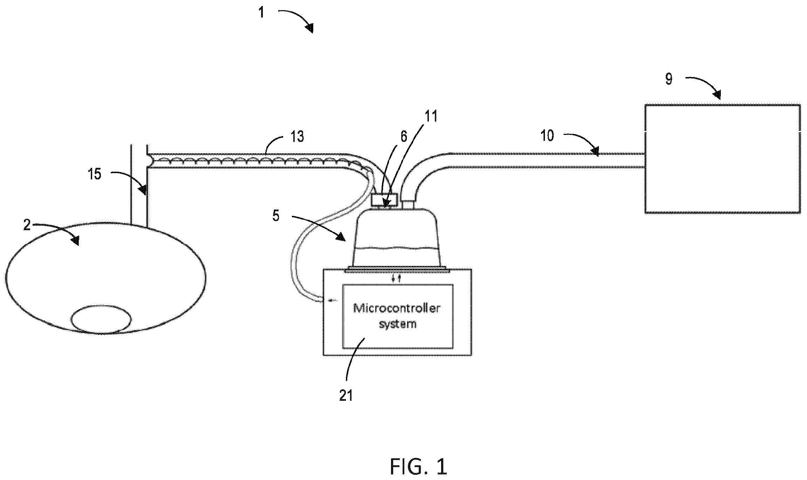

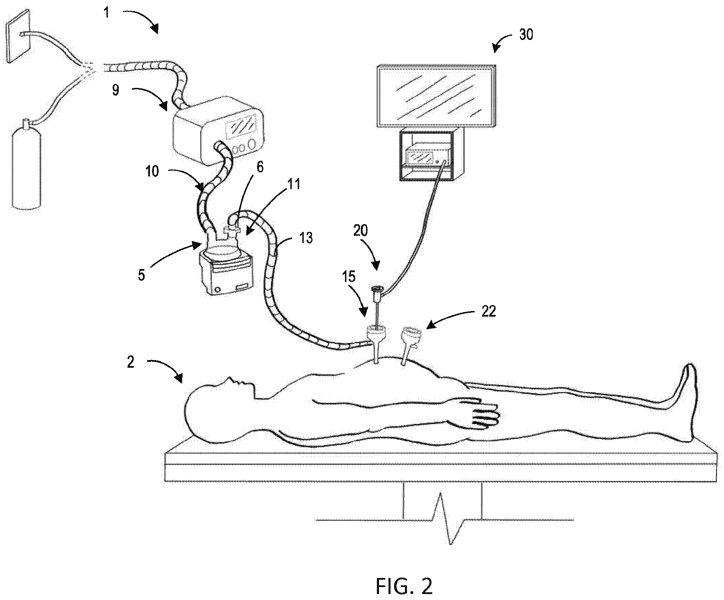

Figures 1 and 2 illustrate schematically using an example surgical, for example, insufflation system 1 during a medical procedure. Features of Figures 1 and 2 can be incorporated into each other. The same features have the same reference numerals in Figures 1 and 2. As shown in Figure 1, the patient 2 can have a cannula 15 inserted to access a cavity of the patient 2 (for example, an abdomen of the patient 2 in the case of a laparoscopic surgery), as previously described.

As shown in Figures 1 and 2, the cannula 15 can be connected to a gases delivery conduit 13 (for example, via a Luer lock connector 4). The cannula 15 can be used to deliver gases into a surgical site, for example, within the cavity of the patient 2. The cannula 15 can include one or more passages to introduce gases and/or one or more medical instruments 20 into the surgical cavity. The medical instrument can be a scope, tool for electrocautery, electro-surgery, energy and laser cutting and/or cauterizing, among others, or any other instrument. The medical instrument 20 can be coupled to an imaging device 30, which can have a screen. The imaging device 30 can be part of a surgical system, which can include a plurality of tools and/or apparatuses. While the application of fluids, for example gases, surrounding the medical instrument is described as important for visualization of the surgical area by a scope or other visualization device, the application of fluids as described herein can be used in applications such as electrocautery tools, graspers, and other instruments.

The system can also optionally include a venting cannula 22, which can have substantially the same features as the cannula 15 and which can be coupled to a filtration system to filter out smoke and the like. The gases delivery conduit 13 can be connected to a humidifier, such as a humidifier with a humidifier chamber 5. The humidifier chamber 5 can be in fluid communication with a gases supply 9 via a further conduit 10. The humidifier may include a controller or control unit 21, which can include a microprocessor for controlling supply of energy to a heating element of the humidifier. A filter 6 can be connected downstream of the humidifier's outlet 11 or upstream of the humidifier inlet. The filter can also be located along the further conduit 10, or at an inlet of the cannula 15. The humidifier and components of the system can be any type suitable for use in medical procedures, such as the Fisher and Paykel Healthcare Humigard SH870 surgical humidifier and the ST320 humidified insufflation kit for laparoscopic and open surgery.

As described herein, a proximal direction with respect to an obturator or cannula generally can refer to the top end of the cannula or obturator body. A distal direction generally can refer to the bottom end of the obturator or cannula shaft configured to be the first section of the trocar assembly inserted into the surgical cavity. As described herein, a proximal direction with respect to a medical instrument generally can refer to the top end of the medical instrument body, while a distal direction with respect to a medical instrument generally can refer to the bottom end of the medical instrument body configured to be the first section of the medical instrument inserted into the cannula and/or surgical cavity. Reference numerals of the same or substantially the same features may share the same last two digits.

### Examples of Cannulas

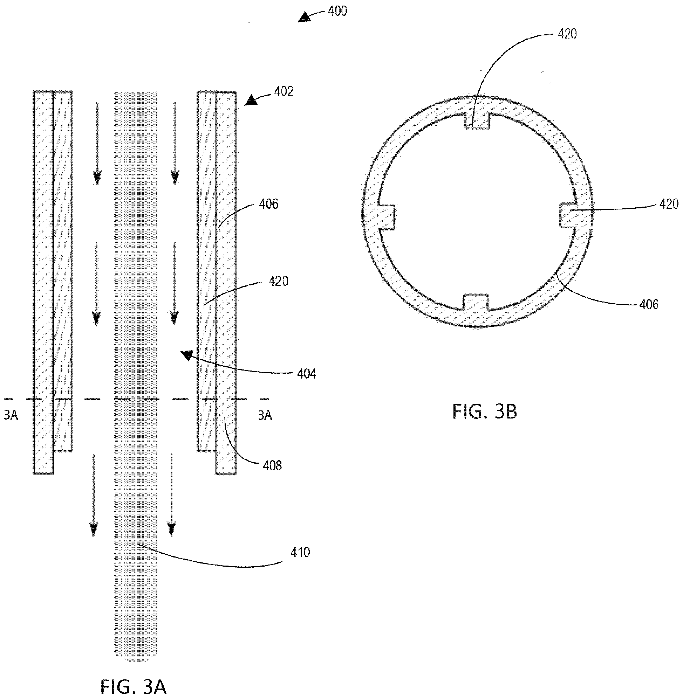

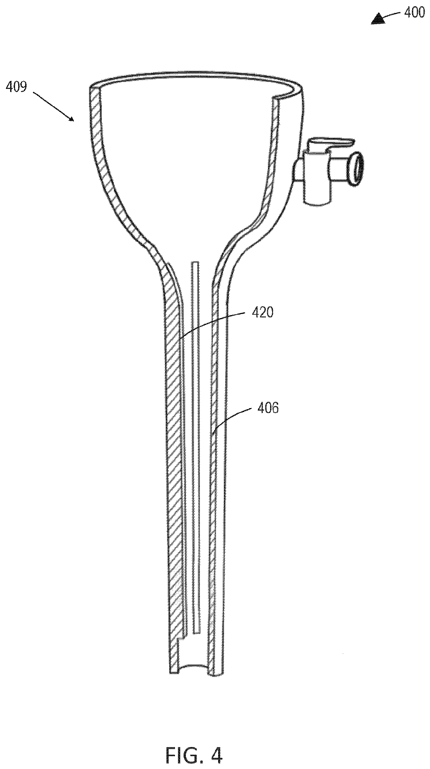

The cannula 400 shown in Figures 3A-3B and 4 illustrates an example cannula that may be used during the aforementioned types of surgery. The cannula 400 comprises a top housing/body 402 and an elongate shaft 406 extending from the top housing 409. The cannula 400 may have internal structures such as guide elements 420, for example ribs, that may be configured to locate a medical instrument 410, such as a scope, within the cannula shaft. For example, the guide elements 420 may be configured to position the medical instrument 410 substantially concentrically with respect to a longitudinal axis of the cannula shaft and/or body. The internal structures may be located at one location or at multiple locations along the shaft 406; that is, in a longitudinal or axial direction along the shaft 406. Slight angled deviations with respect to the axial axis of the cannula shaft are also envisioned. For example, the medical instrument can be between 0 to 30 degrees off axis with respect to the cannula shaft. The guide elements 420 may be integrally formed with or removably coupled to the inner wall 408 of the cannula body and/or shaft. The guide elements 420 or similar structures protruding outwardly from the cannula inner wall 408 may be present for other purposes, such as directing vented gases from the patient cavity or for heating the gases. In a non-limiting example, the guide elements 420 may be configured to provide the desirable effect of preventing or at least reducing contaminants and/or unwanted debris from depositing onto a viewing structure of the medical instrument, for example the lens of a laparoscope.

The guide elements 420 may be configured to assist in preventing or at least reducing condensation that may form on or drip onto a viewing surface that could result in impairment of vision or a 'fogging effect'. Similarly, non-water based particles such as smoke particles in or around the viewing surface may impair vision. The guide elements 420 can be configured to substantially direct the gases along the medical instrument 410 and/or beyond the distal end of the medical instrument such that the gases create an environment adjacent to and/or around the lens to achieve the two example effects described above. However, the guide elements 420 located in the cannula may obstruct other medical instruments from being inserted through the lumen 404 in the cannula shaft, such as an obturator for example. It would be desirable then, that the obturator be compatible with the cannula that it is used with. Similar problems may apply where there is at least one region and/or feature located on and/or within the cannula shaft and/or body for example, bumps, fins, dimples or any other structure. One advantage of such an obturator is that it provides compatibility across a range of cannula types, that is, cannulas with and without 'guide elements' or other similar such internal structures.

The arrangement of the medical instrument 410 within the cannula shaft 406, for example where the medical instrument 410 is positioned substantially concentrically with respect to the longitudinal axis of the cannula shaft 406, promotes or encourages maintained contact of the insufflation gases with the instrument, e.g. a lens of a scope. This contact may also allow for defogging of or clearing of smoke or other particles from the instrument, in particular the lens. In general, increasing the flow rate of the insufflation gases can reduce the required defogging/clearing time. Cold dry gas provided to the cannula while the instrument is held within the cannula shaft can also help to defog or clear the lens. The defogging/clearing can be improved with warming of gases. Warming of the gas can be achieved using a humidifier such as the SH870 humidifier from Fisher & Paykel Healthcare (Auckland, NZ) which can further humidify the gases.

More detailed examples of the cannula features or guide elements that the obturator described herein are designed to be compatible with are described in International Application No. PCT/NZ2019/050100, titled "DIRECTED GAS FLOW SURGICAL CANNULA FOR PROVIDING GASES TO A PATIENT," filed on August 16, 2019.

### Examples of Obturator for use with Cannulas

An obturator with features compatible with cannula having internal structural elements or features (for example, the cannulas described with reference to Figures 4A-4E and having one or more guide elements) can be used in combination with the cannulas with internal features described herein. As used herein, the cannula can have one or more guide elements or other similar structures protruding outwardly from the inner wall, that is, inwardly towards a centre of the cannula shaft 406. As used herein, the cannula with internal structures can be referred to as a cannula generally and the cannula can include one or more internal structures, features or elements. The obturator and the cannula can be used together or bundled as a trocar. As used herein, a trocar can be referred to as an obturator-cannula assembly and these terms can be used interchangeably. The cannula can be used with a gas supply (and/or a humidification system) in the surgical field. The obturator can assist the cannula to be inserted into the patient and therefore can be used whenever a cannula is used.

The cannulas described herein have internal structures, such as guide elements, which, as discussed, can interfere with the obturator. Therefore, the obturator described herein can be designed to be compatible with these types of cannula.

When the obturator is used with the cannula the obturator can be designed to prevent gas from leaking out or around the obturator. The obturator can be removably coupled with the cannula. In some cases, a medical instrument, e.g. a scope, can be inserted and retained in an obturator that allows optical instrumentation or visualization. In some cases, the medical instrument can visualize through the tip of an obturator that has an optically clear or transparent material. As used herein, an optical version of the obturator or an optical obturator can refer to an obturator that has an optically clear or transparent material at its tip to allow visualization through the tip, for example to contribute to optical clarity and clear vision for the medical instrument at the surgical cavity. The obturator-cannula assembly can be fit for the purpose of inserting the cannula into the patient. The obturator-cannula assembly can be used to gain entry into the patient's body and/or for reentry, for example, to gain access again after removal of a tissue sample.

The obturator can have features configured to be compatible with the cannula with structural features disposed on and/or within an inner surface of the cannula shaft and/or body to avoid interference with or damage of the internal structures. The obturator shaft and/or tip can have a region or feature that accommodates the internal structures without compromising their function. In some cases, as used herein, the internal structures can be guide elements as described herein or other internal structures within the cannula.

In some cases, the obturator can have features to accommodate other features on the inner surface of the cannula, for example features that create turbulence as well as instrument retaining elements e.g. dimples, outwardly extending walls or other protrusions.

### Tip of Obturator

The obturator can have a variety of tips on the penetrating distal end of the shaft. The tip of the obturator can be designed to be received by the cannula without causing damage to the internal structures located in the cannula or the tip itself. This can allow the obturator to be compatible with a cannula with internal structures. In some cases, the obturator can be compatible with a range of different cannula types. For example, the obturator can be compatible with cannulas with and without internal structures, such as guide elements.

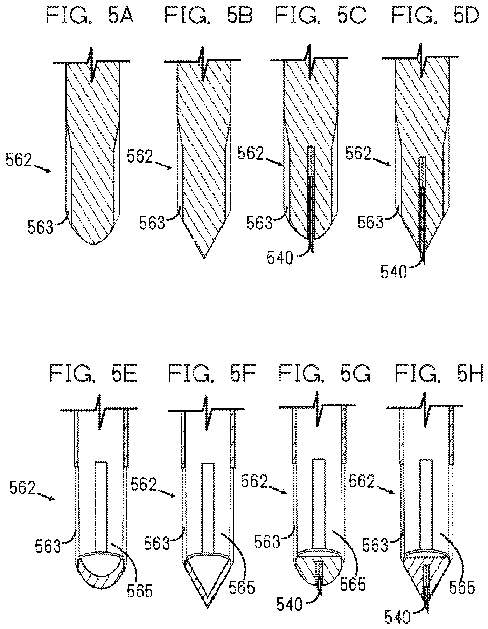

Figures 5A-5P illustrate embodiments of obturator tip variations that can be used with the different embodiments of the obturator described herein. The obturator may have any one of the tip variations and may accommodate the internal structures of the cannula regardless of the tip variation. The obturator tip can be integrally formed with or removably coupled to the obturator shaft. The obturator shaft may therefore include the tip at its distal end. The obturator tip can be made from a different material to the obturator shaft and/or the tip itself may be made from more than one material. The obturator tip can be removably coupled to the obturator shaft to enable different embodiments of the internal structure accommodating portions to be attached and accommodate differently shaped and/or configured internal structures.

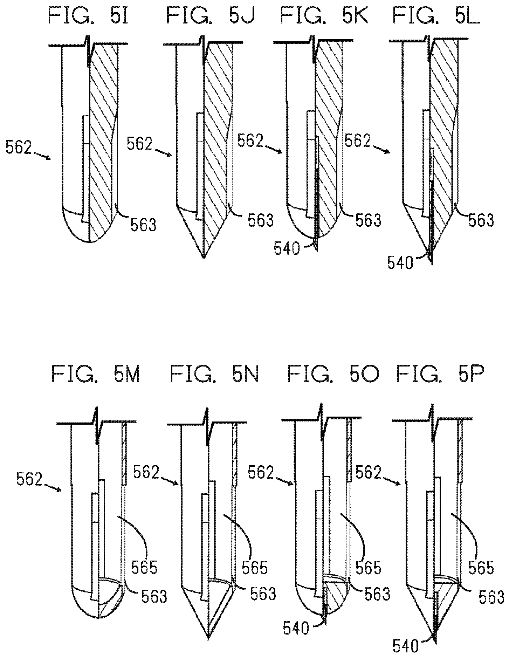

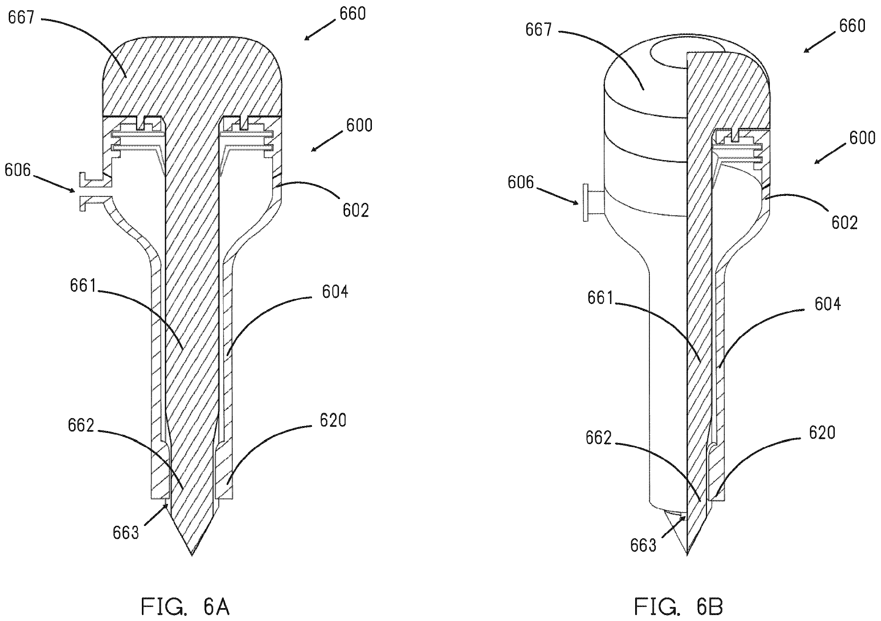

The obturator tip can have a penetrating end at the distal end. The variations of the tip shown in Figures 5A-5P include three categories of penetrating ends at the tip, creating a plurality of possible tip combinations. These combinations can include a blunt tip, dilating tip, bladed tip, non-bladed tip, optical and non-optical tip. As shown in Figures 5A-5P, the obturator tips 562 can include at least one internal structure accommodating portion 563 such as, for example, a recess, an opening or slot as described below with reference to Figures 6A-7D. The recesses, openings or slots are shown to illustrate how the recesses, openings or slots could be integrated into any of the eight tip variations. Figures 5A-5H illustrate cross-sectional views of embodiments of obturator tip variations. Figures 5I-5P illustrate partial slice views of embodiments of obturator tip variations. Figures 5A and 5I illustrate a blunt tip non-optical obturator. A blunt tip can be a non-sharp tip or a tip that is less sharp than a dilating tip. Figures 5B and 5J illustrate a dilating tip, non-optical obturator. Figures 5C and 5K illustrate a non-optical, blunt tip obturator with a bladed tip 540. Figures 5D and 5L illustrate a dilating bladed tip obturator with a bladed tip 540. Figures 5E-5H and 5M-5P illustrate an optical obturator that includes a lumen 565 that allows a medical instrument, such as a scope, to be inserted into the lumen during use. Figures 5E and 5M illustrate a blunt tip optical obturator. Figures 5F and 5N illustrate a dilating tip optical obturator. Figures 5G and 5O illustrate a blunt tip optical obturator with a bladed tip 540. Figures 5H and 5P illustrate a dilating bladed tip optical obturator with a bladed tip 540.

In some cases, the obturator can have one or more internal structure accommodating portions 563 on the obturator shaft at or adjacent to the obturator tip 562. The position of the one or more internal structure accommodating portions 563 may substantially coincide with the location of the one or more internal structures on the cannula inner wall. In some cases, the region of the obturator shaft which has the internal structure accommodating portions can be removably attached to the obturator shaft. In some cases, different designs of the internal structure accommodating portions could be removably attached to the obturator shaft to be suitable for differently shaped and/or differently positioned cannula internal structures. For example, the cannula may have internal structures at the proximal end of the cannula and/or at the distal end of the cannula or at any position along its length. Accordingly, the internal structure accommodating portions may include one or more recesses, openings or slots at any point along the obturator shaft as well as, or instead of, at or adjacent to the obturator tip 562. The one or more recesses, openings or slots can include multiple discrete internal structure accommodating portions 563 or can include elongate recesses, openings or slots that extend substantially the length of the obturator shaft and/or the distal tip 562

The or each internal structure accommodating portion 563 can provide clearance for the obturator from the internal structures so the obturator can be inserted into the cannula and the obturator shaft received in the cannula lumen. Figures 6A-6B illustrate cross-sectional views of an example embodiment of the obturator 660 within a cannula 600 with internal structures 620. Figures 6A-6B illustrate views of a cannula 600 with a cannula body 602, elongate shaft 604, and a gases inlet 606. The elongate shaft 604 can include a hollow passage. The obturator 660 can have an obturator body 667 at the proximal end of the obturator 660. The obturator 660 can have an obturator shaft 661 that extends from the obturator body 667. The obturator shaft 661 may be configured to be received within the hollow passage of the elongate shaft 604 of the cannula 600. The distal end of the obturator shaft 661 can include the obturator tip 662. The obturator 660 can be positioned within the cannula 600, with the obturator shaft 661 located in the elongate shaft 604 of the cannula 600.

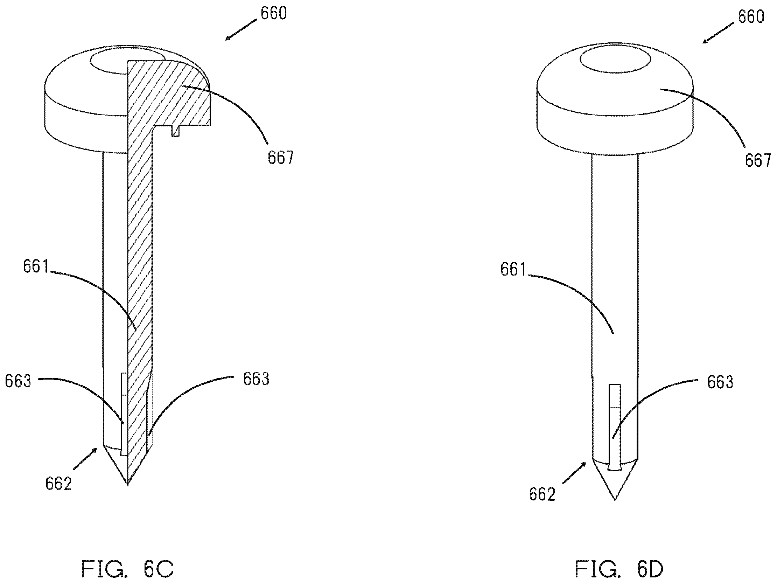

As shown in Figures 6A-6D, the obturator 660 can include at least one internal structure accommodating portion at or adjacent the obturator tip 662. In some embodiments not shown in the Figures, the internal structure accommodating portion or portions can be located anywhere along the elongate shaft 661, for example at its proximal end, distal end or at a mid-point of the obturator shaft 661 or may extend along substantially the entire length thereof. The obturator 660 can include one or more internal structure accommodating portions that correspond to the internal structures on the cannula 600. The internal structure accommodating portion can be one or more recesses, openings or slots 663 as illustrated in Figures 6A-6D. The openings 663 can prevent the obturator 660 from hitting or interfering with (e.g., contacting) the internal structures 620 on the cannula 600. Figure 6C illustrates a view of a slice of an embodiment of an obturator 660 with openings 663 located adjacent the tip 662. The openings 663 may extend from the distal end or tip 662 of the obturator shaft 661, extending at least partway towards the obturator body 667. Figure 6D illustrates a view of an embodiment of the obturator 660 with openings in it which may prevent the obturator 660 from interfering with the internal structures in the cannula 600. As illustrated in Figures 6A-6D, the openings 663 can be recesses or indents in the surface of the obturator shaft 661. The recesses or indents can have sufficient size, configuration, and depth to provide clearance for internal structures 620 in the cannula 600. The openings can be any shape or configuration to accommodate internal structures 620 of different sizes, shapes, and configurations. The internal structure accommodating portion can be any configuration or any positioning that will correspond to and/or be compatible with internal structures 620 on the cannula 600. Although Figures 6A-6D and 8A-12E illustrate a solid obturator body, a hollow body obturator can also be used.

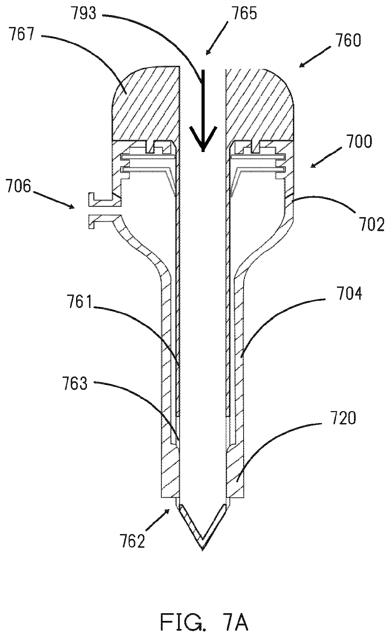

In some cases, for example in the hollow body obturator, the obturator can have a lumen that runs substantially through the length of the obturator. The lumen in the obturator can be defined by a sidewall, such as an inner sidewall of the obturator shaft. The lumen can extend substantially the length of the obturator shaft. The obturator with a lumen running through can be an optical obturator. The lumen can act as a pathway to allow access for a medical instrument, such as a vision accessory, and/or surgical scope. The lumen can create a pathway that can allow access for a viewing instrument such as a scope or camera element to provide visualization. Figure 7A illustrates a cross-sectional view of an embodiment of the obturator 760 within a cannula having internal structures. The cannula 700 may have a cannula body 702, elongate shaft 704, and a gases inlet 706. The elongate shaft 704 can include a hollow passage 708. An obturator 760 can be positioned within the cannula 700. The obturator 760 can have an obturator body 767 at the proximal end of the obturator 760. The obturator 760 can have an obturator shaft 761 that extends from the obturator body 767. The obturator shaft 761 is configured to be receivable within the hollow passage 708 of the elongate shaft 704 of the cannula 700. The obturator 760 can have a lumen 765 that extends from the proximal end of the obturator towards a distal end of the obturator 760. The lumen 765 can be used to allow insertion of a medical instrument, for example a scope. The distal end of the obturator shaft 761 can include the obturator tip 762. The embodiments of the obturator 760 described herein may be shown without a lumen within the obturator, however, it is understood that any of the obturator embodiments described herein can include a lumen running through the obturator from a proximal end towards a distal end of the obturator.

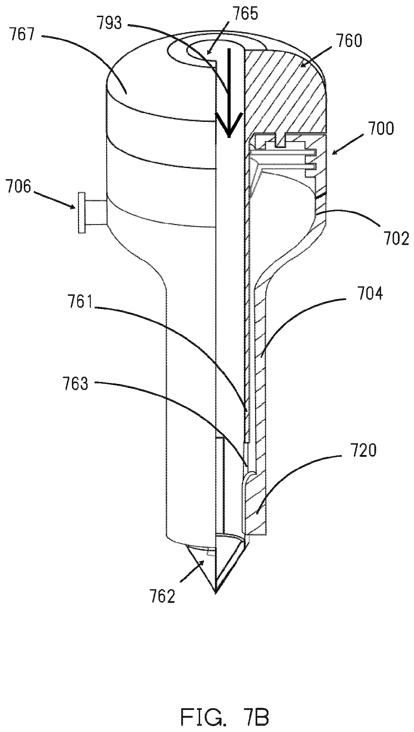

Figure 7B illustrates views of a slice of embodiment of the obturator with a lumen within a cannula with internal structures. Figures 7A shows an optical obturator without a medical instrument inserted in the lumen. In Figure 7B, a medical instrument may be placed in the lumen of the obturator as shown schematically by the arrow 793. As illustrated in Figures 7A-7C, the optical obturator can have a lumen to allow access and positioning of a medical instrument. The internal structure accommodating portions can be openings 763 that are cut-outs that extend through a sidewall of the obturator shaft 761. In contrast, Figures 6A-6D illustrate an obturator where the internal structure accommodating portions are recessed in the obturator shaft 661.

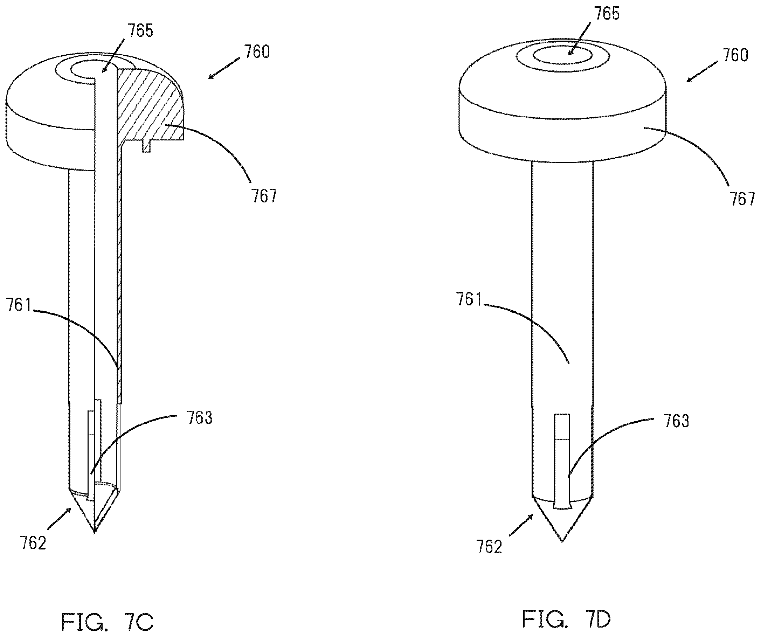

Figure 7C illustrates a view of a slice of an embodiment of an optical obturator with internal structure accommodating portions, such as recesses, openings or slots, which may prevent the obturator from interfering with the internal structures in the cannula. Figure 7D illustrates a view of an embodiment of an optical obturator with openings 763 in the shaft adjacent the obturator tip 762.

The obturator 760 has openings 763 at or adjacent the tip 762. The openings 763 may be configured to coincide with the location of internal structures 720 on the cannula 700. This means that the openings 763 provide clearance for the obturator 760 from the internal structures 720 so the obturator 760 can be inserted into the cannula 700. The openings 763 can provide an opening or recess on the surface of the shaft of the obturator 760. In some cases, the openings 763 can be positioned anywhere in or on the obturator shaft 761, such as at a distance from the tip 762, for example. The openings 763 can also be located at or near the proximal end, distal end or at a mid-point of the obturator shaft 761 or may extend along substantially the entire length thereof.

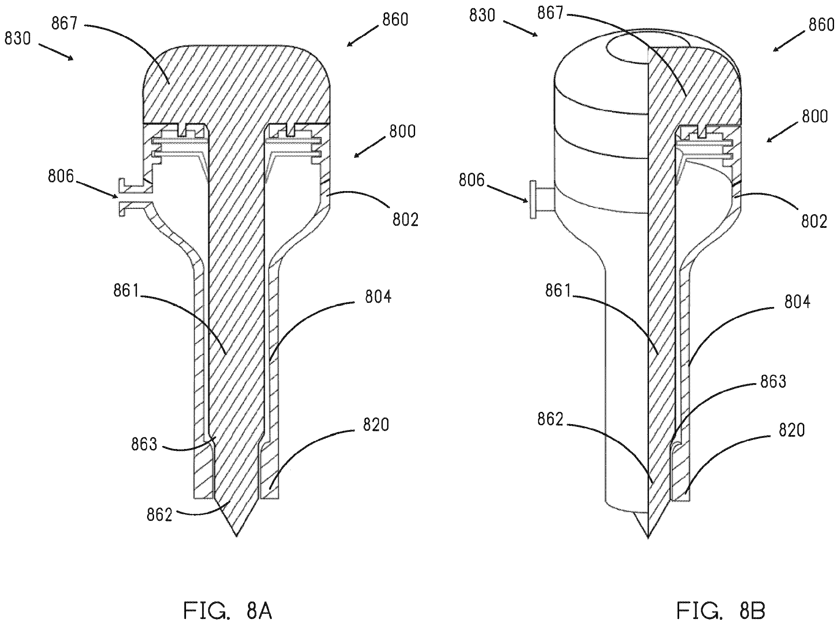

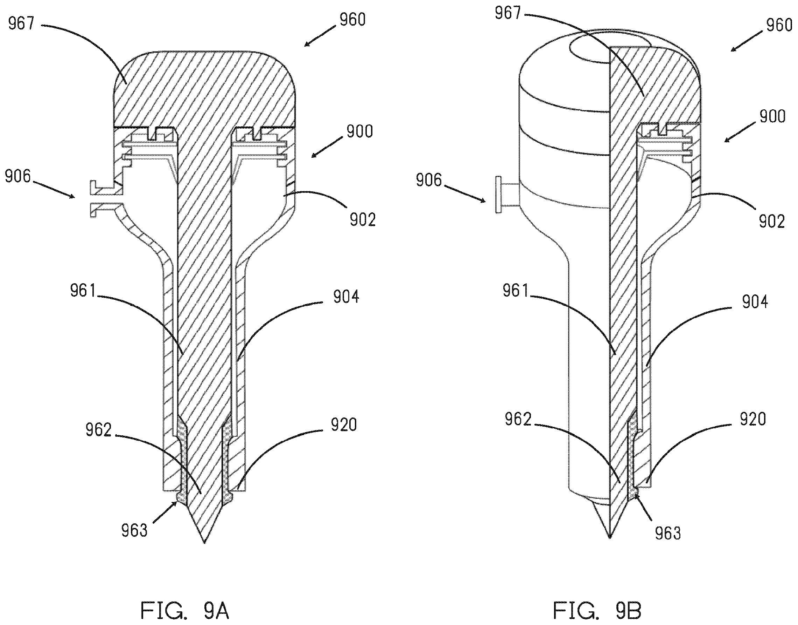

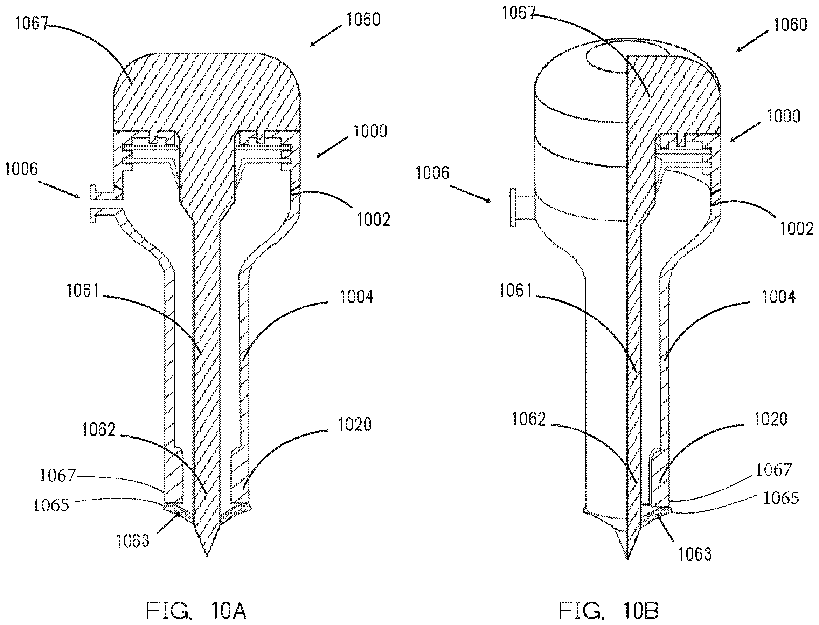

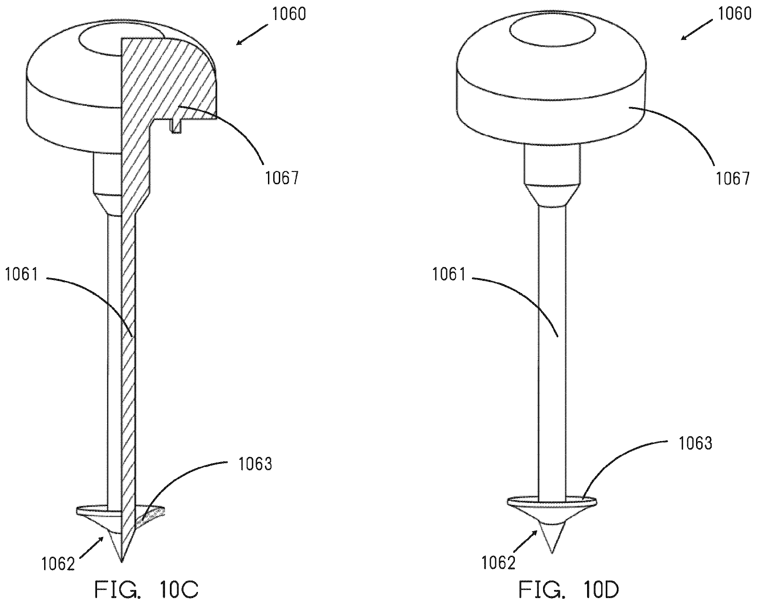

The obturator 760 can have various internal structure accommodating portions that allow the obturator shaft 761 to be inserted into the cannula 700 and allow compatibility of the obturator 760 with a cannula 700 with internal structures. For example, the obturator 760 can include recesses, slots or openings as described in Figures 6A-7D. In other cases, the obturator can have internal structure accommodating portions that can include one or more of an undersized or reduced diameter obturator shaft portion and/or tip (shown in Figures 8A-8D, Figures 16A-16C and Figures 18A-18C), a flexible or deformable material in the obturator shaft and/or tip (shown in Figures 9A-9D), and/or a flexible ring at the obturator shaft and/or tip (shown in Figures 10A-10D). The features described with reference to Figures 6A-10D can be used in combination with the features described with reference to Figures 5A-5P.

Figure 8A illustrates a cross-sectional view of an embodiment of a trocar 830 comprising obturator 860 within a cannula 800 with internal structures 820. Figure 8B illustrates a view of a slice of the obturator 860 within the cannula 800 with internal structures. The cannula 800 of Figures 8A-8B has a cannula body 802, elongate shaft 804, and a gases inlet 806. Additionally and/or alternatively, the cannula 800 may comprise a gases outlet (not shown). The elongate shaft 804 can include a hollow passage. The obturator 860 can be positioned within the cannula 800. The obturator 860 can have an obturator body 867 at the proximal end of the obturator 860. The obturator 860 can have an obturator shaft 861 that extends from the obturator body 867. The obturator shaft 861 may in use, be positioned within the hollow passage of the elongate shaft 804 of the cannula 800. The obturator shaft 861 can be received in the hollow passage of the cannula 800. The distal end of the obturator shaft 861 can include the obturator tip 862. The distal end of the obturator 860 and/or the obturator tip 862 can have a reduced diameter portion relative to the remainder of the obturator shaft 861. The reduced diameter portion may allow the obturator 860 to fit within the cannula 800 with internal structures 820 at or adjacent the distal end of the cannula 800 as shown in Figures 8A-8B.

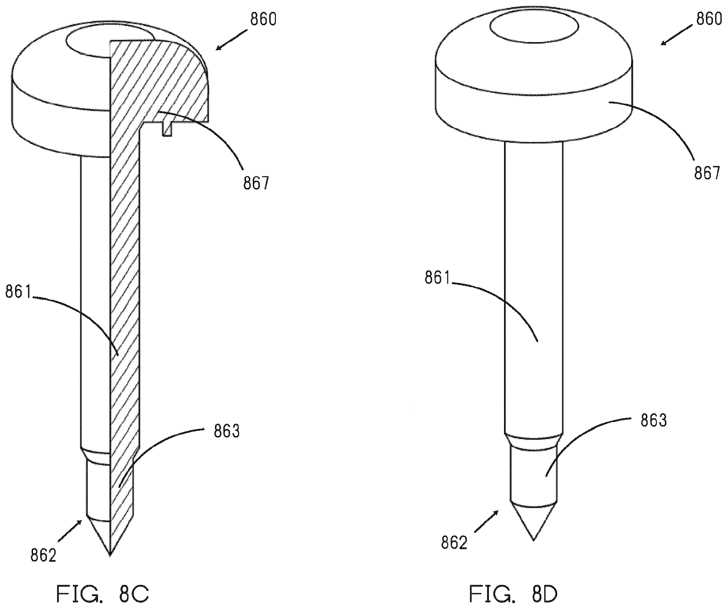

Figure 8C illustrates a view of a slice of the obturator 860 with a reduced diameter portion 863 at or adjacent the distal end of the obturator shaft 861 and/or tip 862. The reduced diameter portion 863 may prevent the obturator 860 from interfering with the internal structures 820 in the cannula 800. Figure 8D illustrates a further view of the obturator 860 of Figure 8C. The reduced diameter portion 863, shown at or adjacent the distal end of the obturator and/or the obturator tip 862 in Figures 8C and 8D, can include a smaller dimension with respect to a proximal portion of the obturator 860, such as a smaller diameter for example. As shown in Figures 8A-8D, the obturator tip 862 can include an undersized (e.g., smaller diameter) section 863 in the obturator tip 862. However, the reduced diameter portion 863 may be located anywhere on the obturator shaft 861, as may be dictated by the position of internal features of the cannula 800, for example at or near the proximal end, distal end and/or at a mid-point of the obturator shaft 863. Furthermore, there may be at least one reduced diameter portion 863 or more than one reduced diameter portion 863 present along the length of the obturator shaft 861.

As used herein, the reduced diameter portion 863 at the distal end of the obturator and/or the obturator tip 862 of the obturator 860 in the embodiment of Figures 8C and 8D can have a diameter that is less than the diameter of other portions of the obturator shaft 861. The reduced diameter portion 863 of the distal end of the obturator and/or obturator tip 862 of the embodiment of Figures 8C and 8D can have a diameter that is less than the inner diameter of the lumen of the cannula 800 it is made for. Further, the one or more reduced diameter portions 863 of the distal end of the obturator and/or obturator tip 862 can have a diameter that is less than the diameter of an imaginary circle that touches the innermost surfaces of the internal structures 820 in the cannula 800. Therefore, although there are internal structures 820 in the cannula 800, the reduced diameter portions of the obturator 860 will not interfere with them. In some cases, an obturator 860 can be sized for the cannula 800 it is to be used with, which may assist ease of insertion of the obturator into the cannula and may, in use, prevent tissue and debris from entering the cannula. In this case, the obturator 860 can be formed with a reduced diameter portion 863 for the internal structures in the cannula 800 so that the obturator 860 does not interfere with the internal structures 820 in the cannula 800.

In some cases, the obturator can have a flexible or deformable material in or on the obturator shaft and/or tip to accommodate the cannula internal structures. The flexible or deformable material may allow for improved sealing of the cannula during insufflation and/or may help prevent blockage or occlusion of the cannula by unwanted media. Figure 9A illustrates a cross-sectional view of an embodiment of the obturator 960 with a deformable material 963 surrounding a portion of the obturator shaft 961 and/or obturator tip 962 within a cannula 900 with internal structures 920. The deformable material can be a sheath around a distal end of the obturator shaft as shown in Figures 9A-9D. Figure 9B illustrates a view of a slice of the obturator 960. Figures 9A-9B illustrate views of the cannula 900 with a cannula body 902, elongate shaft 904, and a gases inlet 906. The elongate shaft 904 can include a hollow passage. The obturator 960 can have an obturator body 967 at the proximal end of the obturator 960. The obturator 960 can have an obturator shaft 961 that extends from the obturator body 967. The obturator 960 can be positioned within the cannula 900, such that the hollow passage of the elongate shaft 904 of the cannula 900 receives the obturator shaft 961. The distal end of the obturator shaft 961 can include the obturator tip 962. The deformable material 963 is located on at least a distal portion of the obturator shaft 961 and/or tip 962 to allow the material of the obturator 960 to flex or deform to fit within the cannula 900 with internal structures 920 as shown in Figures 9A-9B.

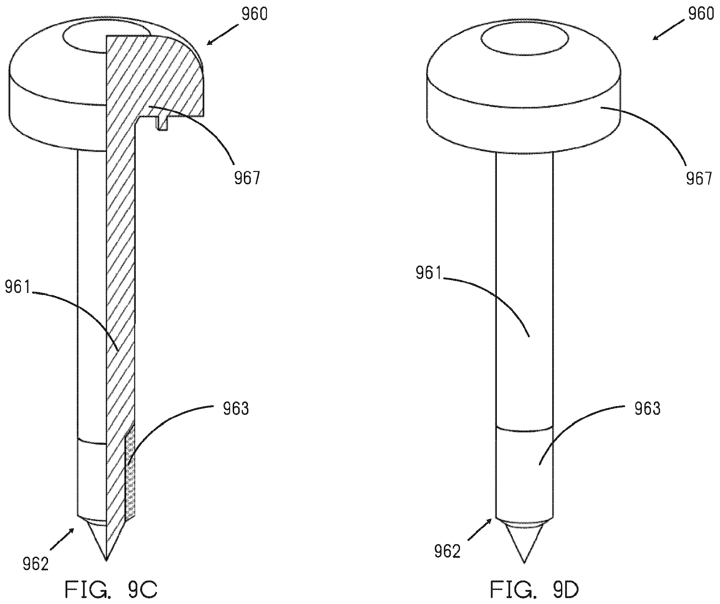

Figure 9C illustrates a view of a slice of the obturator 960 with a deformable material 963 in the obturator shaft and/or tip. Examples of suitable deformable materials for this purpose include natural rubber, synthetic natural rubber, silicone rubbers with additives, silicone, and thermoplastic elastomers (TPE). The deformable material 963 may assist to prevent the obturator 960 from damaging the internal structures 920 in the cannula 900. Figure 9D illustrates a view of the obturator 960 separate from the cannula 900. As shown in Figures 9A-9D, the obturator tip 962 can include a deformable material 963 in the obturator shaft and/or tip. The deformable material 963 of the obturator 960 can deform around the internal structures 920.

The obturator 960 can have a deformable material 963 at the tip 962 to allow the obturator 960 to flex, compress or otherwise deform so as to yield to the internal structures 920 in the cannula 900. The deformable material 963 can allow the obturator 960 to be received by the cannula 900 without interfering with or damaging the internal structures 920 in the cannula 900 and/or the obturator itself. In some cases, as shown in Figures 9A-9B, the tip 962 of the obturator 960 can extend from the distal end of the cannula 900. The deformable material 963 can flex, compress or otherwise deform to yield to the internal structures 920 including covering the distal end of the internal structures 920. The deformable material 963 can assist to seal the cannula 900 to help prevent fluid, tissue and debris entering the cannula 900, such as while it is being inserted into the surgical cavity. The obturator 960 can have a deformable material 963 in the shaft, for example at the midpoint of the shaft, and/or tip of the obturator 960 which can deform around the internal structures 920 in the cannula 900 allowing the obturator 960 to be inserted into and received by the cannula 900.

In some cases, such as the embodiment shown in Figures 10A - 10D, the obturator 1060 can have a flexible structure 1063 located around the obturator shaft 1061and/or tip 1062 to accommodate the internal structures 1020 of the cannula 1000. In some cases, the flexible structure 1063 can be a ring located around the obturator shaft 1061 and/or tip 1062. As shown in Figures 10A-10B, the cannula 1000 has a cannula body 1002, elongate shaft 1004, and a gases inlet 1006. The elongate shaft 1004 can include a hollow passage with one or more internal structures 1020 arranged on an inner surface of the hollow passage. The obturator 1060 can be configured to be positioned within the hollow passage of the cannula 1000. In some cases, the obturator 1060 can be slidably engagable with the hollow passage of the cannula 1000. The obturator 1060 can have an obturator body 1067 at the proximal end of the obturator 1060. The obturator 1060 can have an obturator shaft 1061 that extends from the obturator body 1067. The obturator shaft 1061 may be configured to be received within the hollow passage of the elongate shaft 1004 of the cannula 1000. The distal end of the obturator shaft 1061 can include the obturator tip 1062. The obturator shaft 1061 and/or obturator tip 1062 can have one or more of the flexible structures 1063 thereon to allow the obturator 1060 to fit within the cannula 1000 with internal structures 1020 at the distal end of the cannula 1000. In some embodiments, the one or more flexible structures 1063 can be located anywhere along the obturator shaft 1061, for example at or near the proximal end, distal end and/or at a mid-point of the obturator shaft 1061.

The obturator tip 1062 can include a flexible structure 1063 disposed about the obturator shaft and/or tip. The flexible material 1063 can prevent the internal structures 1020 in the cannula 1000 from interfering with the obturator 1060.

The flexible structure 1063 at the tip 1062 of the obturator 1060 can flex, compress or otherwise deform as it passes over the internal structures 1020 of the cannula 1000 as the obturator shaft 1061 is inserted into the cannula 1000. Once the obturator 1060 is positioned in the cannula shaft to form the trocar assembly, the flexible structure 1063 can spring open or otherwise return to its pre-flexed, pre-compressed or pre-deformed state to cover the tip of the cannula 1000. For example, an outer edge 1065 of the flexible ring may lie adjacent to an outer edge 1067 of the distal end of the cannula 1000. Once the flexible structure 1063 passes over the internal structures 1020, the flexible structure 1063 can be located at the distal end of the cannula 1000 with its outer edge 1065 lying adjacent the outer edge 1067 of the cannula distal end. Since the diameter of the obturator 1060 is smaller than the diameter of the inner lumen of the cannula 1000, a gap created between the obturator 1060 and the inner sidewall of the cannula lumen can allow for debris to enter the cannula lumen. The flexible structure 1063 can be located at the distal end of the cannula 1000 and can create a seal, covering the distal end of the cannula 1000. This can help to prevent fluid, tissue, and debris from entering the cannula 1000 as the obturator and cannula are inserted into the surgical cavity. Preventing fluid, tissue and debris from entering the cannula 1000 can be advantageous in some cases to prevent this fluid, tissue, and debris from sticking or otherwise attaching to the cannula internal structures 1020 and potentially reducing their function. In some cases, the flexible structure 1063 can provide for smoother insertion of the obturator 1060 and can reduce the likelihood of tissues, organs, or other substances getting stuck in the gaps between the obturator and inner wall of cannula. The obturator 1060 can be removed from the cannula 1000 by pulling it back up through the cannula 1000. The flexible structure 1063 can flex out of the way of the internal structures 1020. The obturator 1060 thus can have a flexible structure 1063 at or adjacent the tip 1062 which can deform on contacting the internal structures 1020 during insertion into the cannula shaft and/or removal from the cannula 1000. The flexible structure 1063 can physically contact the internal structures 1020 as the obturator 1060 is passed through the lumen of the cannula 1000.

### Locating Elements of Obturator

The obturator may need to be in a certain orientation before it is attached to the cannula. The obturator may have one or more locating elements to locate the obturator onto the cannula and ensure that it is orientated correctly. Proper orientation of the obturator can prevent the obturator from damaging internal structures in the cannula or itself during engagement with the cannula.

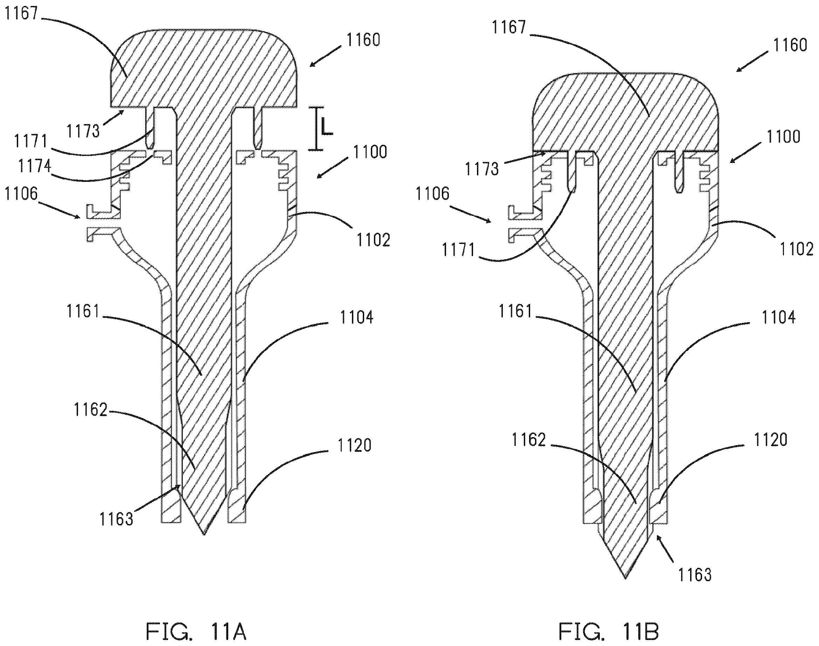

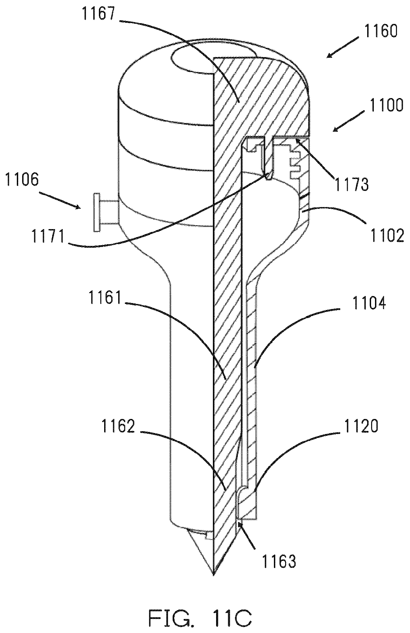

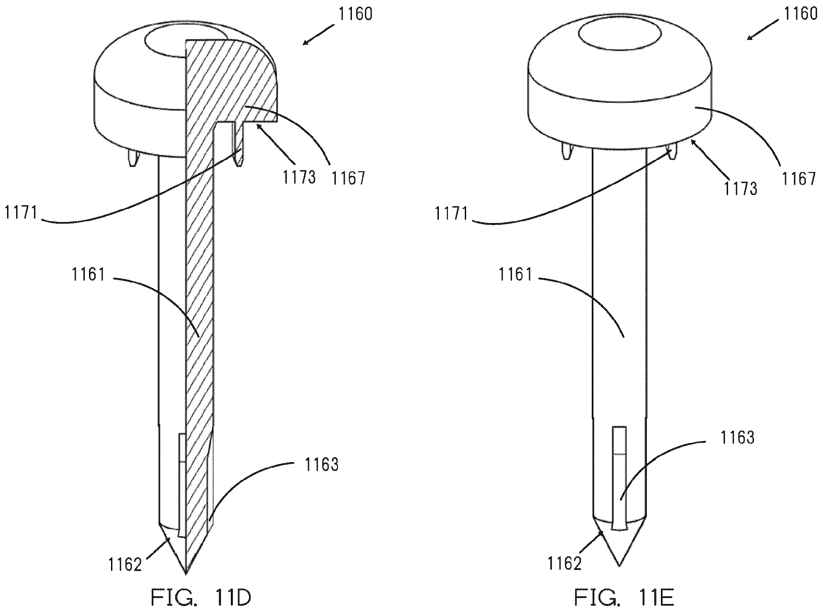

In some cases, such as shown in Figures 11A - 11D, the obturator 1160 can have one or more locating elements 1171. The one or more locating elements 1171 may be at a proximal end of the obturator 1160. The locating elements 1171 may assist to appropriately orient the obturator 1060 within the cannula 1100. Figures 11A-11B illustrate cross-sectional views of embodiments of the obturator 1160 with locating elements 1171 configured to be positioned within a cannula 1100, such as a cannula with internal structures 1120. The obturator 1160 and cannula 1100 shown in Figures 11A-11E can be similar to the obturator and cannula described with reference to Figures 6A-10D and can have similar features. Additionally, the features described with reference to Figures 5A-10D can be used in combination with the features described with reference to Figures 11A-11E.

Figures 11A-11C illustrate views of the cannula 1100 with a cannula body 1102, elongate shaft 1104, and a gases inlet 1106. The elongate shaft 1104 can include a hollow passage. The obturator 1160 can be positioned within the cannula 1100. The obturator 1160 can have an obturator body 1167 at the proximal end of the obturator 1160. The obturator 1160 can have an obturator shaft 1161 that extends from the obturator body 1167. The obturator shaft 1161 is configured to be received within the hollow passage of the elongate shaft 1104 of the cannula. The distal end of the obturator shaft 1161 can include the obturator tip 1162. The obturator tip 1162 and/or obturator shaft 1161 can incorporate any of the features described herein to accommodate the internal structures 1120 in the cannula 1100, for example, the internal structure accommodating portions 1163. The proximal end of the obturator 1160 can include the obturator body 1167 that is positioned over the proximal end of the cannula body 1102 when the obturator 1160 is inserted within the cannula 1100. One or more locating elements 1171 can be located on a cannula contacting surface 1173 of the obturator body 1167. The locating elements 1171 can be a downwardly depending member such as a protrusion, tab or pin structure that extends distally from the cannula contacting surface 1173 of the obturator 1160. That is, the locating element 1171 depends downwardly from the cannula contacting surface 1173 as shown in Figures 11A-11E, parallel to the longitudinal axis of the obturator shaft 1161. The locating elements 1171 can have a length L. The proximal end of the cannula 1100 can include complementary receiving elements 1174 in the cannula body 1102. The receiving elements can be a size and shape that can receive the locating elements 1171 as the obturator 1160 is inserted into and in engagement with the cannula 1100 as shown in Figures 11A-11C. The locating elements 1171 can include one or more pins extending from the underside or cannula contacting surface 1173 of the obturator body 1167. In some cases, the locating elements 1171 can assist to orient the obturator 1160 to the cannula 1100 before the cannula internal structures 1120 contact the obturator 1160. The length (L) of the locating elements 1171 can be sufficient to allow for this to happen.

As shown in Figures 11A-11E, the obturator 1160 can have locating elements 1171 on the body 1167 of the obturator 1160 to help align the obturator 1160 axially to the cannula 1100. This may assist to make sure that the obturator 1160 has its features, in particular the internal structure accommodating portions, aligned with the internal structures 1120 of the cannula 1100 before being fully assembled. If the obturator 1160 is not aligned properly then the internal structures or guide elements 1120 of the cannula 1100 or the obturator 1160 itself could be damaged. Whilst the obturator shaft 1161 or its tip 1162 is the first portion of the obturator 1160 to contact the cannula 1100, the locating elements 1171 are the first structures to align with the cannula 1100 when being inserted. The locating elements 1171 may also help reduce torsional stress between the ribs or other internal structures of the cannula and recesses, slots or openings of the obturator during insertion into the patient. In some cases, the locating elements 1171 can be long enough (have length L) to ensure that the obturator 1160 does not interfere with the internal structures 1120 in the cannula before it is properly orientated.

As illustrated in Figures 11A-11E, the obturator 1160 can have locating elements 1171, for example, pins, that are long enough to ensure that the locating elements orient the obturator to the cannula correctly before the internal structures in the cannula contact the obturator. This can prevent the obturator from being inserted in the wrong orientation.

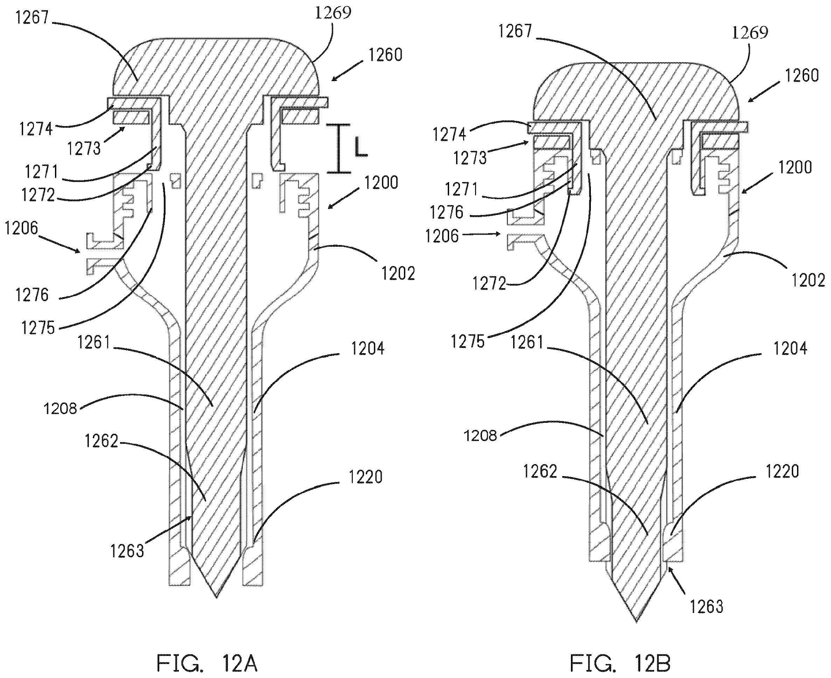

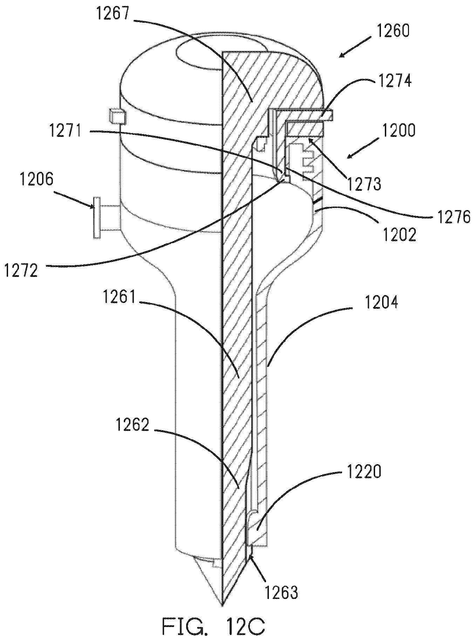

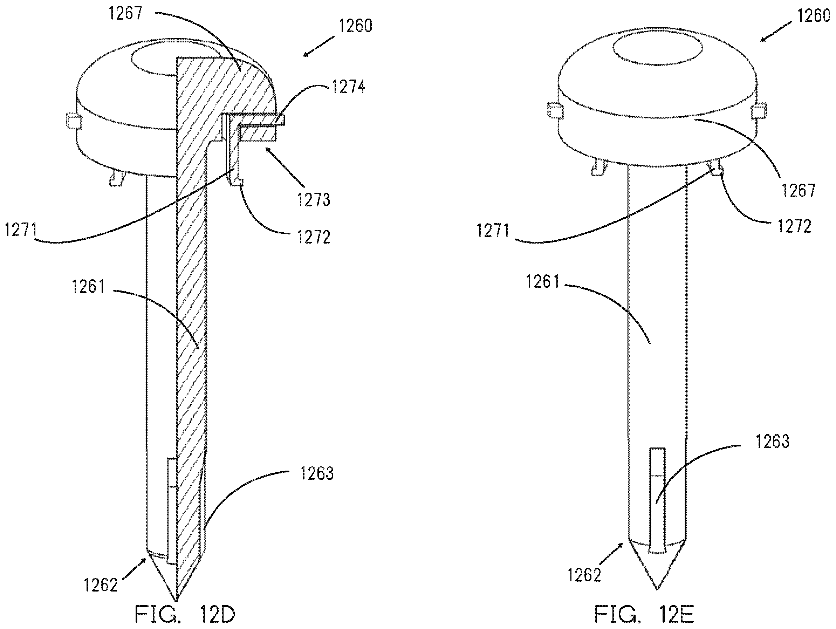

In some cases, the obturator can include a locating element 1271 on the obturator body 1267 at a proximal end of the obturator. The locating element can clip into or otherwise engage the cannula to properly orient the obturator 1260 axially within the cannula 1200, to assist with aligning the internal structure accommodating portions 1263 of the obturator with the internal structures of the cannula 1200 before it is fully inserted into the cannula. In some cases, one or more locating elements can be used that clips the obturator into the cannula. Figures 12A-12B illustrate cross-sectional views of embodiments of the obturator 1260 with locating elements that clip the obturator 1260 into a cannula 1200 with internal structures 1220 to help retain the obturator 1260 inside the cannula 1200. The obturator 1260 and cannula 1200 shown in Figures 12A-12E can be similar to the obturator and cannula described with reference to Figures 6A-10D and can have similar features. Additionally, the features described with reference to Figures 5A-10D can be used in combination with the features described with reference to Figures 12A-12E.

Figures 12A-12C illustrate views of a cannula 1200 with a cannula body 1202, elongate shaft 1204, and a gases inlet 1206. The elongate shaft 1204 can include a hollow passage 1208. The obturator 1260 can be positioned within the cannula 1200. The obturator 1260 can have an obturator body 1267 at the proximal end of the obturator 1260. The obturator 1260 can have an obturator shaft 1261 that extends from the obturator body 1267. The obturator shaft may be configured to be received within the hollow passage 1208 of the elongate shaft 1204 of the cannula 1200. The distal end of the obturator shaft 1261 can include the obturator tip 1262. The obturator tip 1262 and/or obturator shaft 1261 can incorporate any of the features described herein to accommodate the internal structures 1220 in the cannula 1200, for example, the internal structure accommodating portions 1263. The proximal end of the obturator 1260 can include the obturator body 1267. The obturator body 1267 may be positioned over the proximal end of the cannula body 1202 when the obturator 1260 is inserted within the cannula 1200.

One or more locating elements 1271 may extend from a cannula contacting surface 1273 of the obturator body 1267 or may extend from other parts of the obturator body 1267, for example its outer surface 1269. In the embodiment shown in Figures 12A-12C, the locating elements 1271 are in the form of locking finger locating elements 1271. The locking finger locating elements 1271 can be a locking finger structure that is incorporated into the body 1267 of the obturator. The locking finger structure may extend distally from the cannula contacting surface 1273 of the obturator. The locking finger locating element 1271 can have a release structure 1274. The release structure 1274 can extend outwardly from an upper end of the locking finger locating element 1271 and substantially perpendicularly thereto so as to form an inverted L-shaped structure with the downwardly depending part of the locking finger locating element 1271. The release structure 1274 extends radially through a horizontal channel in the cannula body 1267 such that it protrudes from the outer surface 1269 of the obturator body 1267. In the embodiment shown in Figures 12A - 12C, the obturator 1260 has two of the locking finger locating elements 1271 disposed diametrically opposite one another on the obturator body 1267. The protruding portions of the release structures 1274 can be pressed inward to move the locking finger locating elements 1271 to release the obturator 1260 from the cannula 1200.

A portion of the locking finger locating elements 1271 that extends downwardly from the cannula contacting surface 1273 can have a length L. The length L can be long enough to ensure that the obturator 1260 does not interfere with the internal structures in the cannula 1200 before it is properly orientated. The proximal end of the cannula 1200 can include corresponding apertures 1275 that are a size and shape that can receive the locking finger locating elements 1271 when the obturator is engaged with the cannula as shown in Figures 12B-12C. In some cases, the locating elements can include at least one locking finger extending outwardly, i.e. distally from the lower surface of the cannula contacting surface 1273 of the body 1267 and the locking finger(s) can be configured to be received in the corresponding apertures 1275 in the cannula body 1202. In some cases, the locking fingers 1271 have a locking tab 1272 extending substantially perpendicularly from the lowermost end of the locking finger locating element 1271 and can be configured to engage with a corresponding engagement tab 1276 in the cannula body. As the obturator is engaged with the cannula the locking tab 1272 of the locking finger locating element 1271 can flex or pivot around the corresponding engagement tab 1276 in the cannula body to lock the obturator in the correct orientation within the cannula. As the release structure 1274 is pressed inwardly, the locking finger locating element 1271 can move or flex inwardly to move the locking tab 1272 around the corresponding engagement tab 1276 and thereby releasing the obturator from the cannula.

As shown in Figures 12A-12E, the obturator 1260 can have locking finger locating elements on the body of the obturator 1260 to help align the obturator 1260 axially to the cannula 1200. The locating elements can assist to align features of the obturator 1260, in particular the internal structure accommodating portions 1263, with the internal structures 1220 of the cannula 1200 before being fully assembled as a trocar. If the obturator 1260 is not aligned properly then the internal structures 1220 in the cannula 1200 or the obturator 1260 itself could be damaged. In some cases, the locking finger locating elements can be long enough (have length L) to ensure that the obturator 1260 does not interfere with the internal structures 1220 in the cannula 1200 before it is properly orientated.

The obturator 1260 can have a locking finger locating element 1271 that clips into the proximal end of the cannula to help retain the obturator 1260 inside the cannula. The locking finger locating element 1271 can ensure that the obturator 1260 and cannula will not come apart during use. Once the cannula has been inserted as required for the surgical procedure, the release structures 1274 on the side can be pressed which allows the obturator 1260 to be removed from the cannula.

The obturator 1260 can have locking finger locating elements 1271 that are long enough to ensure that the clips orient the obturator 1260 to the cannula correctly before the internal structures in the cannula contact the obturator 1260. This may prevent the obturator being inserted in the wrong orientation. The locking finger locating elements 1271 can clip or otherwise engage into the proximal end of the cannula which may help retain the obturator 1260 inside the cannula.

### Examples of Optical Obturator that Prevents Gas Leaking around the Medical Instrument

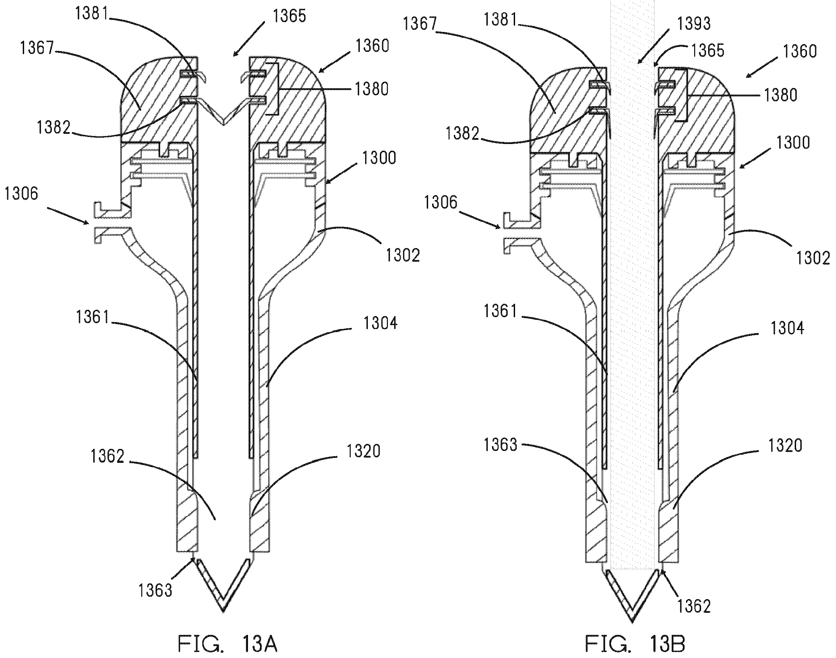

As described previously, an optical obturator can include a lumen that runs longitudinally through the obturator from an opening in the proximal body towards a distal end of the shaft or the tip. In such embodiments, gas may leak out of the obturator. For example, gas can leak out of an opening in the body of the obturator, where the medical instrument, e.g. surgical scope, enters the lumen of the obturator. The obturator can include one or more seal elements to prevent or mitigate such gas leak, such as the example embodiments shown in Figures 13A - 14E. The obturator and cannula shown in Figures 13A-13E can be similar to the obturator and cannula described with reference to Figures 6A-12E and can have similar features. Additionally, the features described with reference to Figures 5A-12E can be used in combination with the features described with reference to Figures 13A-13E.

Figures 13A-13E illustrate an embodiment of an optical obturator 1360 with one or more seal elements 1380. The obturator 1360 with the lumen 1365 can be similar to previously described obturators with a lumen, such as those described with reference to Figures 7A-7D. The lumen can allow for medical instruments to enter and be received by the obturator. The lumen can allow access for vision accessories (e.g. surgical scope) as the cannula and obturator are inserted into and through tissue. The visualization can be achieved by use of a medical instrument, e.g. a scope, that is positioned in the obturator lumen as the cannula and obturator pass through tissue.

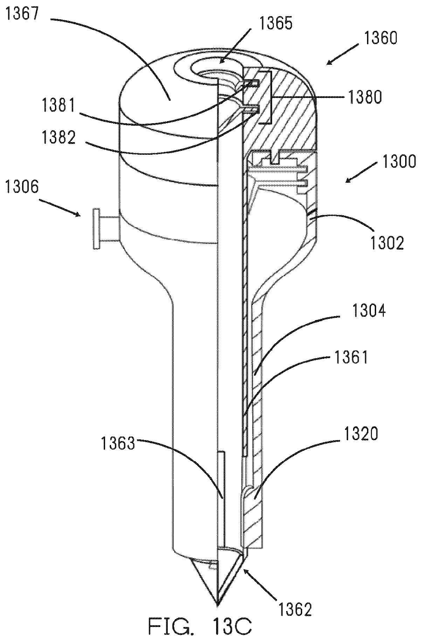

Figures 13A-13B illustrate cross-sectional views of embodiments of the obturator within a cannula with internal structures. Figure 13A shows an optical obturator without a medical instrument inserted in the lumen. Figure 13B illustrates a medical instrument 1393 in the lumen of the obturator. Figure 13C illustrates a view of a slice of an embodiment of the obturator within a cannula with internal structures. Figures 13A-13B illustrate views of a cannula 1300 with a cannula body 1302, elongate shaft 1304, and a gases inlet 1306. The elongate shaft 1304 can include a hollow passage. The obturator 1360 can be positioned within the cannula 1300. The obturator 1360 can have an obturator body 1367 at the proximal end of the obturator 1360. The obturator 1360 can have an obturator shaft 1361 that extends from the obturator body 1367. The obturator shaft 1361 can be configured to be received within the hollow passage of the elongate shaft 1304 of the cannula 1300. The obturator 1360 can have a lumen 1365 that extends from the proximal end of the obturator 1360 towards a distal end of the obturator 1360. The lumen 1365 can allow medical instruments (for example, a surgical scope) to be received by and positioned within the obturator 1360. The distal end of the obturator shaft 1361 can include the obturator tip 1362.

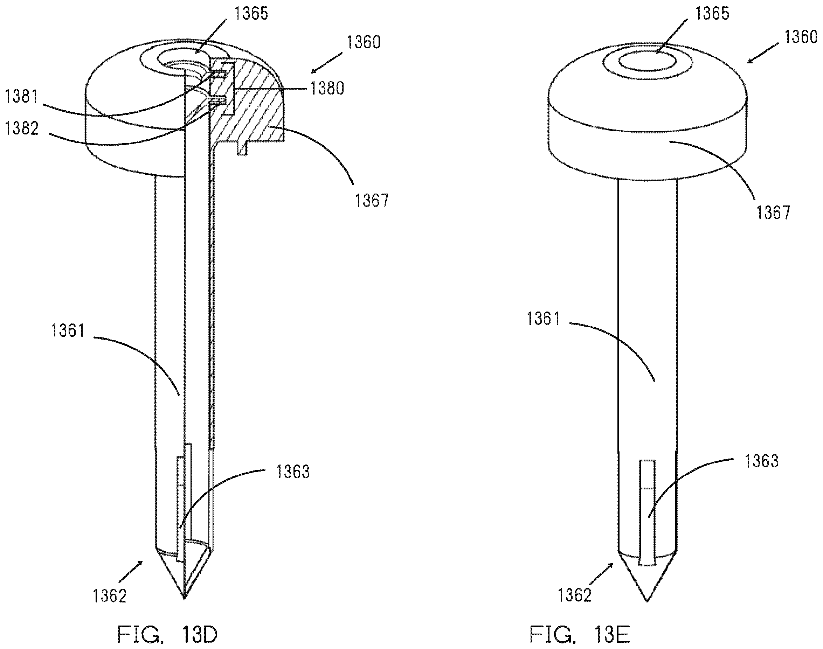

Figure 13D illustrates a view of a slice of an embodiment of an obturator 1360 with a lumen 1365 and with internal structure accommodating portions, for example recesses, openings or slots, 1363 in the obturator shaft, which may prevent the obturator from interfering with the internal structures in the cannula. Figure 13E illustrates a view of an embodiment of an obturator with a lumen 1365 and internal structure accommodating portions, for example recesses, openings or slots, 1363 which may prevent the obturator from interfering with the internal structures in the cannula. As shown in Figures 13A-13E, the obturator shaft 1361 and/or tip 1362 can include internal structure accommodating portions in the outer surface of the shaft 1361, tip 1362, and/or adjacent the tip 1362 to accommodate the internal structures 1320 on the cannula. In some cases, the internal structure accommodating portions can be, for example, openings 1363. The openings 1363 can prevent the obturator from interfering with the internal structures 1320 in the cannula. However, the openings 1363 and the lumen 1365 can create a path for gas to leak out of the obturator 1360. The obturator 1360 can have one or more seal elements 1380 at the proximal end to prevent gas escaping through the lumen. Gas has the potential to enter the lumen of the obturator 1360 if the obturator 1360 has openings or other similar structures that cut into the shaft. The openings can be similar to the openings described with reference to Figures 6A-7D. The distal end of the medical instrument 1393 can be located at the opening of the distal end of the obturator corresponding to the internal structure accommodating portions. In some cases, the distal end of the medical instrument 1393 can be substantially level with the tip.

The one or more seal elements 1380 can include an upper seal 1381 and a lower seal 1382. The upper seal 1381 can seal around a medical instrument, for example a scope, when the medical instrument is inserted into the obturator 1360. The lower seal 1382 can create a gas seal for example when there is no medical instrument inserted in the obturator 1360.

The obturator 1360 can have a seal element 1380 with one or more seals in the proximal end of the obturator. The seal element 1380 can be located in the obturator body 1367. The seal element 1380 can be located in or on the inner sidewall of the lumen 1365 in the obturator body 1367. The seal elements 1380 can extend inwardly into the lumen 1365 of the obturator 1360. The seal elements 1380 can create an annular sealing ring (as shown in Figure 13D) around the opening in the obturator body 1367. The seal elements 1380 can be used to seal the optical obturator 1360 which may have openings which can create a gas pathway from the cannula into the obturator 1360.

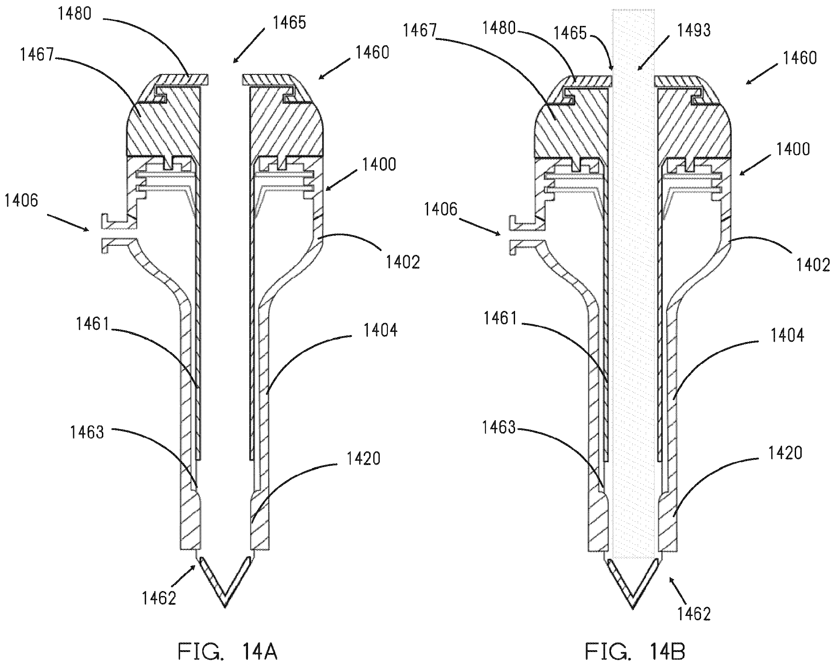

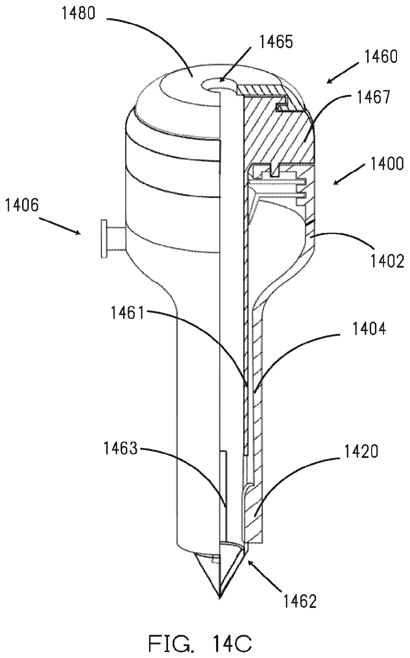

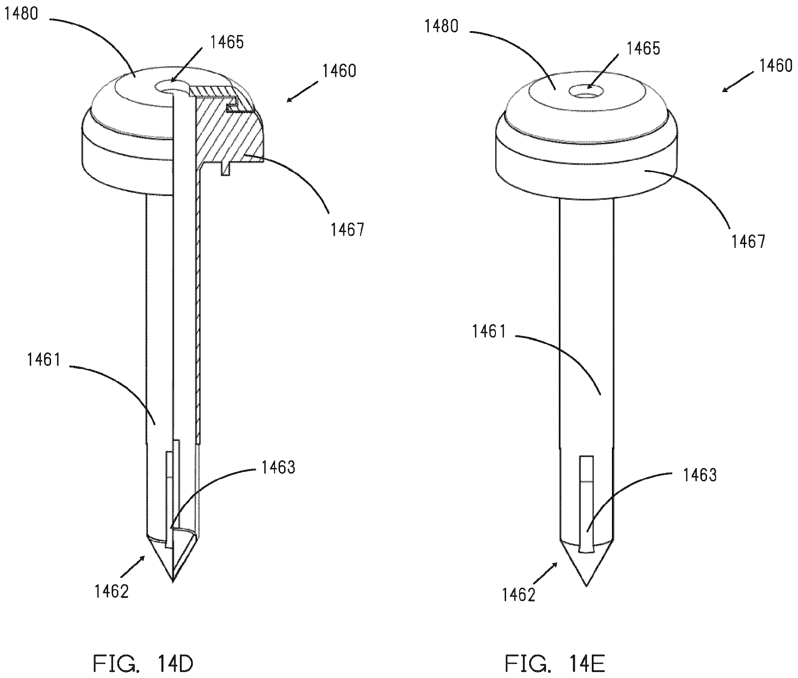

Figures 14A-14E illustrate another embodiment of an obturator 1460 with one or more seal elements 1480 that can prevent gas from escaping through the lumen 1465 of the obturator 1460. The obturator 1460 and the cannula 1400 in Figures 14A-14E are similar to the obturator 1360 and cannula 1300 described with reference to Figures 13A-13E. However, the embodiment of Figures 14A-14E utilize one or more proximal seals 1480. The obturator and cannula shown in Figures 14A-14E can be similar to the obturator and cannula described with reference to Figures 6A-12E and can have similar features. Additionally, the features described with reference to Figures 5A-12E can be used in combination with the features described with reference to Figures 14A-14E.

Figures 14A-14B illustrate cross-sectional views of embodiments of the obturator with one or more proximal seals 1480, the obturator positioned within a cannula with internal structures. Figure 14A shows an optical obturator without a medical instrument inserted in the lumen. Figure 14B illustrates a medical instrument 1493 in the lumen of the obturator.

The one or more proximal seals 1480 can be positioned on the proximal end of the obturator body 1467 to prevent gas escaping through the lumen 1465 of the obturator 1460. The proximal seal 1480 can be an annular seal or ring or any other suitable seal that is positioned on the proximal most surface of the obturator body 1467. The proximal seal 1480 can be an annular seal or ring around the proximal opening of the lumen 1467 in the obturator 1460. In some cases, the proximal seal 1480 can have an inner diameter that is smaller than the inner diameter of the lumen 1465 of the obturator 1460. Gas has the potential to enter the lumen 1465 if the obturator 1460 has slots, recesses or other similar openings in the obturator shaft. The one or more seals 1480 can be a flexible or deformable material which can be removably attached to the proximal end of the obturator 1460. The one or more seals 1480 can provide a seal around a medical instrument, for example a scope, when the medical instrument is inserted into the obturator 1460. In some cases, the flexible or deformable material can fit over a top or proximal surface of the obturator body 1467.

In some cases, the one or more seals 1480 at the proximal end of the obturator prevents gas leaking out of the proximal opening of the lumen 1465.

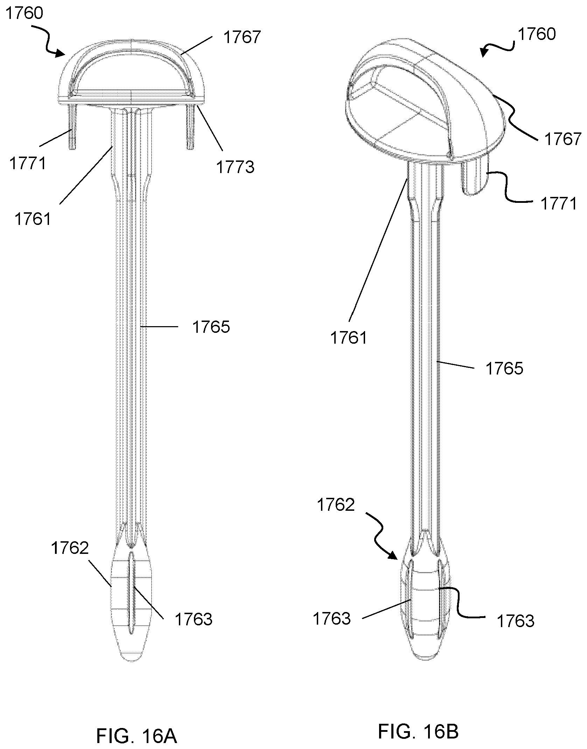

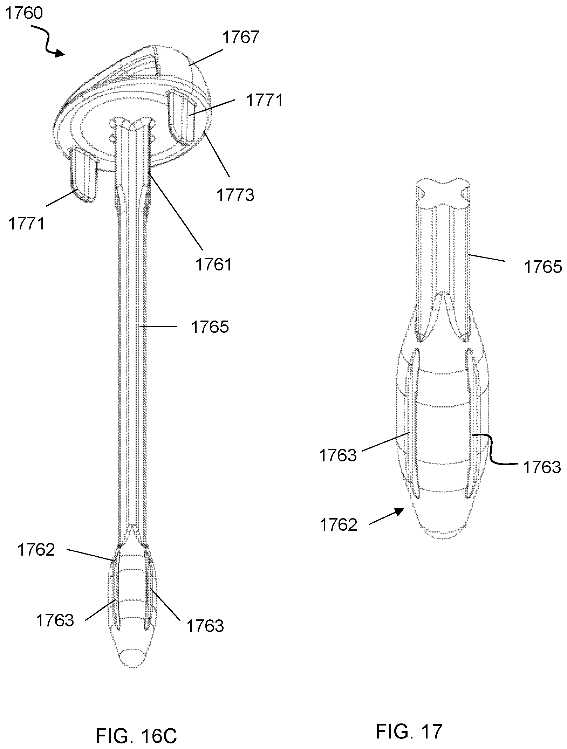

Figures 16A-16C illustrate an embodiment of an obturator 1760 having an obturator body 1767, an obturator shaft 1761 and a blunt distal tip 1762. The obturator shaft 1761 has a cruciform or cross-shaped cross-section. The obturator shaft 1761 has a reduced diameter portion 1765 extending substantially the length of the shaft 1761 between a proximal portion of the shaft 1761 and the distal tip 1762. At the proximal end of the shaft 1761, the shaft diameter tapers gradually to the reduced portion 1765. The reduced diameter portion 1765 of the obturator shaft 1761 reduces friction of the obturator 1760 with the cannula seals as it is inserted into the cannula. The proximal portion of the obturator shaft 1761, above the reduced diameter portion, helps guide the obturator 1760 in place when it is inserted into the cannula and therefore may function as a locating element. The distal tip 1762 is shown more clearly in Figure 17. The distal tip 1762 is elongate in the axial direction of the obturator 1760 and has a blunt, rounded tip. The distal tip 1762 may have a generally elongate oval shape with a maximum diameter that is comparable with an inner diameter of the cannula into which the obturator 1760 will be used, to prevent tissue or other material from ingressing between the obturator and the cannula as they are inserted through tissue. The maximum diameter of the distal tip 1762 may be similar to the diameter of the proximal portion of the obturator shaft 1761. A proximal region of the distal tip 1762 can be tapered with the obturator shaft 1761 to reduce friction against the cannula seals when the obturator is removed from the cannula.

The obturator 1760 has at least one internal structure accommodating portion located thereon. In Figures 16A-16C and Figure 17, the internal structure accommodating portions 1763 are located at the distal tip 1762 and are elongate slots, openings or recesses that extend substantially the majority of the length of the distal tip 1762. The elongate slots, openings or recesses 1763 function to guide the obturator 1760 into the cannula and to receive the guide elements of the cannula as the obturator 1760 is inserted into place. In the illustrated embodiment, four of the elongate slots, openings or recesses are shown, approximately equi-spaced around a circumference of the distal tip 1762. However, any number of the slots, openings or recesses may be present, as may be required to match the guide elements of a particular cannula with which the obturator 1760 is to be used. The accommodating portions 1763 may be located at any point along the obturator shaft 1761, in addition to or instead of at the distal tip 1762.

The obturator body 1767 includes a cannula contacting surface 1773. The obturator 1760 can include at least one locating element 1771 that extends downwardly from the cannula contacting surface 1773. The locating element 1771 can clip into or otherwise engage the cannula or interact with the cannula features to properly orient the obturator 1760 within the cannula. In some embodiments, a plurality of locating elements can be used that clip the obturator into, or otherwise engage with, the cannula. In other embodiments, the locating elements may be dimensioned to fit in e.g. slots on the cannula but may not touch the cannula directly. However if the obturator is twisted, then the location of the locating elements within the slots may prevent it from twisting further. The locating elements 1771 of the embodiment of Figures 16A-16C are in the form of alignment tabs but may also be protrusions, pin structures or other form of locating element. The locating elements 1771 can have a rounded distal end to allow ease of insertion into the cannula and to reduce the likelihood of them snagging or catching on other surfaces, for example surgical gloves. Locating elements having a rounded distal end such as the locating elements 1771 may be used on any of the embodiments of the obturators described herein.

Figures 18A-18C illustrate an embodiment of an obturator 1860 having an obturator body 1867, an obturator shaft 1861 and a blunt distal tip 1862. The obturator 1860 is identical to the obturator 1760 and has each of the features described above, including the reduced diameter portion 1865 of the obturator shaft 1861 and one or more locating elements 1871 extending downwardly from the cannula contacting surface 1873 of the cannula body 1867. The one or more internal structure accommodating portions 1863 also comprise of elongate slots, openings or recesses oriented longitudinally along the distal tip 1862. The slots, openings or recesses 1863 additionally have a flared or splayed portion 1864 at a lower or distal end thereof as best seen in Figure 19. The flared or splayed portion 1864 of the recess, slot or opening 1863 tapers outwardly so as to gradually increase the width of the slot, recess or opening at the lower or distal end of the distal tip 1862. The flared or splayed portion 1864 of the internal structure accommodating portions 1863 assist in guiding the guide elements of the cannula into position as the obturator 1860 is inserted into the cannula. In contrast to the embodiment of Figures. 11A-11E, in which the locating elements 1171 are the first part of the obturator to align with the cannula, it is the flared or splayed portion 1864 of the recess, slot or opening 1863 of the obturator that contacts the cannula ribs or other internal structures first. That is, the splayed or flared portion 1864 may function as a locating element in the same manner as the locating elements 1171. The lower end of the elongate slots, openings or recesses 1763 of the embodiment of Figures 16A-C and Figure 17 can also provide this function. Nevertheless, the locating elements 1771, 1871 are still present in the obturators 1760, 1860 to reduce torsional stress between the ribs or other guide elements of the cannula and the recesses 1763, 1863 of the obturators 1760, 1860.

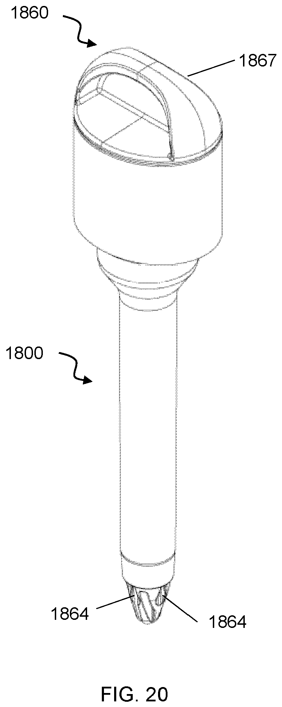

Figure 20 illustrates the obturator 1860 fully inserted into a cannula 1800 to form a trocar. The flared or splayed portions 1864 are shown protruding from a distal end of the cannula 1800, whilst the cannula body 1867 rests on top of the proximal end of the cannula 1800.

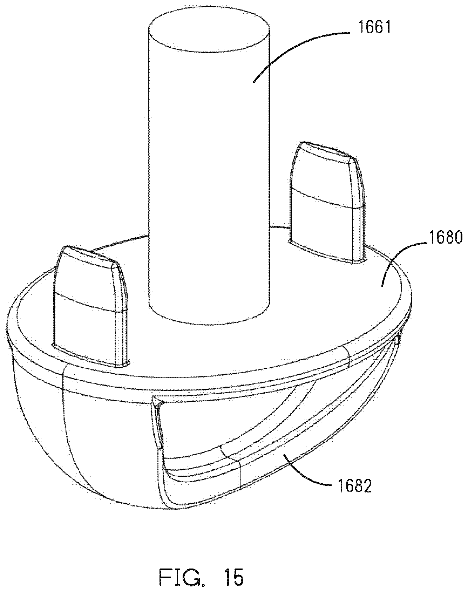

In some cases, any of the obturators described herein can include a handle or grip portion to assist with using the obturator and/or removing the obturator from the cannula. Figure 15 illustrates an example handle 1680 that may be used with any obturator described herein. An obturator shaft 1661 can extend from the handle 1680 (the tip/distal end of the obturator has not been shown for simplicity). The handle 1680 comprises a grip region 1682 which improves usability when removing the obturator from the cannula. In a non-limiting example, the grip region 1682 may provide a surface for a user to apply a force substantially in the same direction as the direction of the removal of the obturator from the cannula. The grip region 1682 may be configured to allow the insertion of a medical instrument (not shown), such as a scope for example.

### Terminology

Examples of obturators and obturator-cannula assembly systems and associated components and methods have been described with reference to the figures. The figures show various systems and modules and connections between them. The various modules and systems can be combined in various configurations and connections between the various modules and systems can represent physical or logical links. The representations in the figures have been presented to clearly illustrate the principles and details regarding divisions of modules or systems have been provided for ease of description rather than attempting to delineate separate physical embodiments. The examples and figures are intended to illustrate and not to limit the scope of the inventions described herein. It will be apparent to the skilled person that one or more features of the various described embodiments may be readily used in combination with one or more features of other described embodiments where appropriate.

Examples described herein illustrate a cannula and/or obturator used in combination with and supporting a scope. However, in some cases, the cannula can be used to hold other medical instruments. Additionally, as referred to herein the terms "concentric", "concentrically", and/or "substantially concentric" or any variations of these terms can also refer to minor axis offsets between the cannula and medical instrument. In some case, for example, the axis offsets can include 0-30 degrees offset.

Although certain embodiments and examples are disclosed herein, inventive subject matter extends beyond the specifically disclosed embodiments to other alternative embodiments. Thus, the scope of the claims or embodiments appended hereto is not limited by any of the particular embodiments described herein. For example, in any method or process disclosed herein, the acts or operations of the method or process can be performed in any suitable sequence and are not necessarily limited to any particular disclosed sequence. Various operations can be described as multiple discrete operations in turn, in a manner that can be helpful in understanding certain embodiments; however, the order of description should not be construed to imply that these operations are order dependent. Additionally, the structures described herein can be embodied as integrated components or as separate components. For purposes of comparing various embodiments, certain aspects and advantages of these embodiments are described. Not necessarily all such aspects or advantages are achieved by any particular embodiment. Thus, for example, various embodiments can be carried out in a manner that achieves or optimizes one advantage or group of advantages as taught herein without necessarily achieving other aspects or advantages as can also be taught or suggested herein.

Conditional language used herein, such as, among others, "can," "could," "might," "may," "e.g.," and the like, unless specifically stated otherwise, or otherwise understood within the context as used, is generally intended to convey that certain embodiments include, while other embodiments do not include, certain features, elements and/or states. Thus, such conditional language is not generally intended to imply that features, elements and/or states are in any way required for one or more embodiments. As used herein, the terms "comprises," "comprising," "includes," "including," "has," "having" or any other variation thereof, are intended to cover a non-exclusive inclusion. For example, a process, method, article, or apparatus that comprises a list of elements is not necessarily limited to only those elements but may include other elements not expressly listed or inherent to such process, method, article, or apparatus. Also, the term "or" is used in its inclusive sense (and not in its exclusive sense) so that when used, for example, to connect a list of elements, the term "or" means one, some, or all of the elements in the list. Conjunctive language such as the phrase "at least one of X, Y and Z," unless specifically stated otherwise, is otherwise understood with the context as used in general to convey that an item, term, etc. may be either X, Y or Z. Thus, such conjunctive language is not generally intended to imply that certain embodiments require at least one of X, at least one of Y and at least one of Z each to be present. As used herein, the words "about" or "approximately" can mean a value is within 10%, within ±5%, or within ±1% of the stated value.

It should be emphasized that many variations and modifications may be made to the embodiments described herein, the elements of which are to be understood as being among other acceptable examples. All such modifications and variations are intended to be included herein within the scope of this disclosure. The scope of protection is defined by the claims. Further, nothing in the foregoing disclosure is intended to imply that any particular component, characteristic or process step is necessary or essential.

## Claims

1. A trocar assembly comprising:
a cannula (15;400;600; 700; 800; 900; 1000; 1100; 1200; 1300; 1800) comprising a shaft with a lumen, and one or more internal structures disposed on an interior wall of a shaft of the cannula; and
an obturator (1860) comprising:
an obturator body (1867);
an obturator shaft (1861) extending from the body and comprising a penetrating end at a distal end thereof, the obturator shaft being at least partially positionable within the cannula;
the obturator shaft comprising at least one internal structure accommodating portion (1863) that correspond to the one or more internal structures on the shaft of the cannula, wherein the at least one internal structure accommodating portion (1863) comprises a recess located in a surface of the obturator shaft **characterized in that** the recess includes a flared portion at a distal end thereof;
wherein the one or more internal structures of the cannula (15;400;600; 700; 800; 900; 1000; 1100; 1200; 1300; 1800) comprise one or more ribs.

2. The trocar assembly of Claim 1, wherein the at least one internal structure accommodating portion (1863) comprises a portion of the obturator shaft comprising a reduced diameter relative to the diameter of the remainder of the obturator shaft.

3. The trocar assembly of Claim 1 or Claim 2, wherein the at least one internal structure accommodating portion (1863) is located at or adjacent to the distal end of the obturator shaft.

4. The obturator of any one of Claims 1 to 3, wherein the at least one internal structure accommodating portion (1863) is located substantially along the length of the obturator shaft.

5. The trocar assembly of any one of claims 2, or 3 or 4 when dependent on claim 2, wherein the reduced diameter portion extends substantially the length of the obturator shaft between a proximal end of the shaft and the distal end of the obturator shaft.

6. The trocar assembly of any one of Claims 1 to 5, wherein the obturator body has a locating element (1871) configured to mate with a corresponding receiving element in the cannula.

7. The trocar assembly of Claim 6, wherein the locating element comprises a protrusion extending from an underside of the obturator body.

8. The trocar assembly of Claim 7, wherein the locating element includes a locking finger extending distally from a lower surface of the obturator body, the locking finger configured to be received in a corresponding aperture in the cannula.

9. The trocar assembly of any one of Claims 1 to 8, wherein the obturator is an optical obturator, comprising a lumen configured to receive a scope.

10. The trocar assembly of Claim 9, wherein the obturator body has a seal element configured to prevent gas leaking from the obturator lumen.

11. The trocar assembly of Claim 10, wherein the seal element is located about the proximal open end of the obturator body, the seal element configured to prevent gas leaking out of the obturator body.

12. The trocar assembly of Claim 11, wherein the seal element comprises a flexible or deformable material configured to form a seal against the medical instrument when inserted into the obturator.

13. The trocar assembly of any one of Claims 1 to 13, wherein the internal structure is at or adjacent the distal end of the cannula.

14. The trocar assembly of any one of Claims 1 to 13, wherein the flared portion of the recess tapers outwardly to gradually increase a width of the recess.

15. The trocar assembly of any one of Claims 1 to 14, wherein the distal end of the obturator shaft is elongate in an axial direction of the obturator and comprises a blunt rounded tip.

## Patentansprüche

1. Trokarbaugruppe, umfassend:
eine Kanüle (15;400;600; 700; 800; 900; 1000; 1100; 1200; 1300; 1800), umfassend einen Schaft mit einem Lumen und eine oder mehrere innere Strukturen, die an einer Innenwand eines Schafts der Kanüle angeordnet sind; und
einen Obturator (1860), umfassend:
einen Obturatorkörper (1867);
einen Obturatorschaft (1861), der sich von dem Körper erstreckt und an einem distalen Ende davon ein eindringendes Ende umfasst, wobei der Obturatorschaft mindestens teilweise innerhalb der Kanüle positionierbar ist;
wobei der Obturatorschaft mindestens einen die innere Struktur aufnehmenden Abschnitt (1863) umfasst, welcher der einen oder den mehreren inneren Strukturen an dem Schaft der Kanüle entspricht, wobei der mindestens eine die innere Struktur aufnehmende Abschnitt (1863) eine in einer Oberfläche des Obturatorschafts positionierte Aussparung umfasst, **dadurch gekennzeichnet, dass** die Aussparung an einem distalen Ende davon einen aufgeweiteten Abschnitt beinhaltet;
wobei die eine oder mehreren inneren Strukturen der Kanüle (15;400;600; 700; 800; 900; 1000; 1100; 1200; 1300; 1800) eine oder mehrere Rippen umfassen.

2. Trokarbaugruppe nach Anspruch 1, wobei der mindestens eine die innere Struktur aufnehmende Abschnitt (1863) einen Abschnitt des Obturatorschafts umfasst, der bezogen auf den Durchmesser des Rests des Obturatorschafts einen reduzierten Durchmesser umfasst.

3. Trokarbaugruppe nach Anspruch 1 oder Anspruch 2, wobei der mindestens eine die innere Struktur aufnehmende Abschnitt (1863) an oder benachbart zu dem distalen Ende des Obturatorschafts positioniert ist.

4. Obturator nach einem der Ansprüche 1 bis 3, wobei der mindestens eine die innere Struktur aufnehmende Abschnitt (1863) im Wesentlichen entlang der Länge des Obturatorschafts positioniert ist.

5. Trokarbaugruppe nach einem der Ansprüche 2 oder 3 oder 4, sofern sie von Anspruch 2 abhängig ist, wobei sich der Abschnitt mit reduziertem Durchmesser im Wesentlichen über die Länge des Obturatorschafts zwischen einem proximalen Ende des Schafts und dem distalen Ende des Obturatorschafts erstreckt.

6. Trokarbaugruppe nach einem der Ansprüche 1 bis 5, wobei der Obturatorkörper ein Positionierelement (1871) aufweist, das konfiguriert ist, um mit einem entsprechenden Aufnahmeelement in der Kanüle zusammenzupassen.

7. Trokargruppe nach Anspruch 6, wobei das Positionierelement einen Vorsprung umfasst, der sich von einer Unterseite des Obturatorkörpers erstreckt.

8. Trokarbaugruppe nach Anspruch 7, wobei das Positionierelement einen Verriegelungsfinger beinhaltet, der sich distal von einer unteren Oberfläche des Obturatorkörpers erstreckt, wobei der Verriegelungsfinger konfiguriert ist, um in einer entsprechenden Öffnung in der Kanüle aufgenommen zu werden.

9. Trokarbaugruppe nach einem der Ansprüche 1 bis 8, wobei der Obturator ein optischer Obturator ist, der ein Lumen umfasst, das konfiguriert ist, um ein Endoskop aufzunehmen.

10. Trokarbaugruppe nach Anspruch 9, wobei der Obturatorkörper ein Dichtungselement aufweist, das konfiguriert ist, um zu verhindern, dass Gas aus dem Obturatorlumen austritt.

11. Trokarbaugruppe nach Anspruch 10, wobei das Dichtungselement um das proximale offene Ende des Obturatorkörpers herum positioniert ist, wobei das Dichtungselement konfiguriert ist, um zu verhindern, dass Gas aus dem Obturatorkörper austritt.

12. Trokarbaugruppe nach Anspruch 11, wobei das Dichtungselement ein flexibles oder verformbares Material umfasst, das konfiguriert ist, um beim Einführen in den Obturator eine Dichtung gegen das medizinische Instrument auszubilden.

13. Trokarbaugruppe nach einem der Ansprüche 1 bis 13, wobei sich die innere Struktur an oder benachbart zu dem distalen Ende der Kanüle befindet.

14. Trokarbaugruppe nach einem der Ansprüche 1 bis 13, wobei sich der aufgeweitete Teil der Aussparung nach außen verjüngt, um eine Breite der Aussparung allmählich zu vergrößern.

15. Trokarbaugruppe nach einem der Ansprüche 1 bis 14, wobei das distale Ende des Obturatorschafts in axialer Richtung des Obturators länglich ist und eine stumpfe, abgerundete Spitze umfasst.

## Revendications

1. Ensemble trocart comprenant :
une canule (15 ; 400 ;600 ; 700 ; 800 ; 900 ; 1000 ; 1100 ; 1200 ; 1300 ; 1800) comprenant un arbre avec une lumière et une ou plusieurs structures internes disposées sur une paroi intérieure d'un arbre de la canule ; et
un obturateur (1860) comprenant :
un corps d'obturateur (1867) ;
un arbre d'obturateur (1861) s'étendant à partir du corps et comprenant une extrémité pénétrante au niveau d'une extrémité distale de celui-ci, l'arbre d'obturateur étant au moins partiellement positionnable à l'intérieur de la canule ;
l'arbre d'obturateur comprenant au moins une partie de réception de structure interne (1863) qui correspond aux une ou plusieurs structures internes sur l'arbre de la canule, dans lequel l'au moins une partie de réception de structure interne (1863) comprend un évidement situé dans une surface de l'arbre d'obturateur, **caractérisé en ce que** l'évidement comporte une partie évasée au niveau d'une extrémité distale de celui-ci ;
dans lequel les une ou plusieurs structures internes de la canule (15 ; 400 ; 600 ; 700 ; 800 ; 900 ; 1000 ; 1100 ; 1200 ; 1300 ; 1800) comprennent une ou plusieurs nervures.

2. Ensemble trocart selon la revendication 1, dans lequel l'au moins une partie de réception de structure interne (1863) comprend une partie de l'arbre d'obturateur comprenant un diamètre réduit par rapport au diamètre du reste de l'arbre d'obturateur.

3. Ensemble trocart selon la revendication 1 ou la revendication 2, dans lequel l'au moins une partie de réception de structure interne (1863) est située au niveau de l'extrémité distale de l'arbre d'obturateur ou adjacente à celle-ci.

4. Obturateur selon l'une quelconque des revendications 1 à 3, dans lequel l'au moins une partie de réception de structure interne (1863) est située sensiblement le long de la longueur de l'arbre d'obturateur.

5. Ensemble trocart selon l'une quelconque des revendications 2, ou 3 ou 4 lorsqu'elle dépend de la revendication 2, dans lequel la partie de diamètre réduit s'étend sensiblement sur toute la longueur de l'arbre d'obturateur entre une extrémité proximale de l'arbre et l'extrémité distale de l'arbre d'obturateur.

6. Ensemble trocart selon l'une quelconque des revendications 1 à 5, dans lequel le corps d'obturateur a un élément de localisation (1871) configuré pour s'accoupler avec un élément de réception correspondant dans la canule.

7. Ensemble trocart selon la revendication 6, dans lequel l'élément de localisation comprend une saillie s'étendant à partir d'une face inférieure du corps d'obturateur.

8. Ensemble trocart selon la revendication 7, dans lequel l'élément de localisation comporte un doigt de verrouillage s'étendant distalement à partir d'une surface inférieure du corps d'obturateur, le doigt de verrouillage étant configuré pour être reçu dans une ouverture correspondante de la canule.

9. Ensemble trocart selon l'une quelconque des revendications 1 à 8, dans lequel l'obturateur est un obturateur optique, comprenant une lumière configurée pour recevoir un endoscope.

10. Ensemble trocart selon la revendication 9, dans lequel le corps d'obturateur a un élément d'étanchéité configuré pour empêcher les fuites de gaz à partir de la lumière d'obturateur.

11. Ensemble trocart selon la revendication 10, dans lequel l'élément d'étanchéité est situé autour de l'extrémité ouverte proximale du corps d'obturateur, l'élément d'étanchéité étant configuré pour empêcher les fuites de gaz hors du corps d'obturateur.

12. Ensemble trocart selon la revendication 11, dans lequel l'élément d'étanchéité comprend un matériau flexible ou déformable configuré pour former un joint d'étanchéité contre l'instrument médical lorsqu'il est inséré dans l'obturateur.

13. Ensemble trocart selon l'une quelconque des revendications 1 à 13, dans lequel la structure interne se trouve au niveau de l'extrémité distale de la canule ou adjacente à celle-ci.

14. Ensemble trocart selon l'une quelconque des revendications 1 à 13, dans lequel la partie évasée de l'évidement s'effile vers l'extérieur pour augmenter progressivement une largeur de l'évidement.

15. Ensemble trocart selon l'une quelconque des revendications 1 à 14, dans lequel l'extrémité distale de l'arbre d'obturateur est allongée dans une direction axiale de l'obturateur et comprend une pointe arrondie émoussée.
